(19) Europäisches Patentamt
European Patent Office
Office européen des brevets

(11) **EP 3 946 000 B1**

(12) **EUROPÄISCHE PATENTSCHRIFT**

(45) Veröffentlichungstag und Bekanntmachung des Hinweises auf die Patenterteilung:
**19.03.2025 Patentblatt 2025/12**

(21) Anmeldenummer: **20716444.3**

(22) Anmeldetag: **31.03.2020**

(51) Internationale Patentklassifikation (IPC):
*A61B 3/10* (2006.01)    *A61B 3/13* (2006.01)
*F21V 8/00* (2006.01)    *G02B 21/06* (2006.01)
*G02B 27/01* (2006.01)    *G02B 27/09* (2006.01)
*G02B 27/10* (2006.01)

(52) Gemeinsame Patentklassifikation (CPC):
**A61B 3/107; G02B 6/0011; G02B 21/0012; G02B 21/084; G02B 27/425; G02B 27/4272**

(86) Internationale Anmeldenummer:
**PCT/EP2020/059118**

(87) Internationale Veröffentlichungsnummer:
**WO 2020/201281 (08.10.2020 Gazette 2020/41)**

(54) **VORRICHTUNGEN ZUM ERZEUGEN VON LEUCHTVERTEILUNGEN MIT LICHTWELLENLEITERN**

DEVICES FOR PRODUCING LUMINOUS DISTRIBUTIONS WITH OPTICAL WAVEGUIDES

DISPOSITIFS POUR LA GÉNÉRATION DE DISTRIBUTIONS DE LUMIÈRE AU MOYEN DE FIBRES OPTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.04.2019 DE 102019108677**

(43) Veröffentlichungstag der Anmeldung:
**09.02.2022 Patentblatt 2022/06**

(73) Patentinhaber: **Carl Zeiss Jena GmbH**
**07745 Jena (DE)**

(72) Erfinder:
• **HILLENBRAND, Matthias**
**07745 Jena (DE)**
• **BUBLITZ, Daniel**
**07646 Rausdorf (DE)**
• **NOBIS, Thomas**
**04315 Leipzig (DE)**
• **HACKER, Martin**
**07745 Jena (DE)**
• **BÜHREN, Tobias**
**89073 Ulm (DE)**
• **KLEINDIENST, Roman**
**99425 Weimar (DE)**
• **PESCH, Alexander**
**07745 Jena (DE)**

(74) Vertreter: **Kraus & Lederer PartGmbB**
**Thomas-Wimmer-Ring 15**
**80539 München (DE)**

(56) Entgegenhaltungen:
WO-A1-2015/167492    WO-A1-2016/149416
WO-A1-2018/204712    US-A1- 2002 149 924
US-A1- 2005 286 266    US-A1- 2014 376 207
US-A1- 2017 052 384    US-A1- 2018 364 409
US-A1- 2020 150 042    US-B2- 10 345 593
US-B2- 10 613 266    US-B2- 7 252 425

**Beschreibung**

Die vorliegende Anmeldung betrifft Vorrichtung zum Erzeugen von Leuchtverteilungen mit Lichtwellenleitern, insbesondere zum Beleuchten von Objekten.

Auf vielen Anwendungsgebieten kann es nützlich sein, Licht, welches von einer Lichtquelle bereitgestellt wird, zu modifizieren, sodass es für verschiedene Anwendungen, insbesondere zum Beleuchten von Objekten, geeignet ist.

Aus der US 8,320,032 B2 sowie der US 2016/0231568 A1 sind Wellenleitersysteme bekannt, die zur Bilderzeugung dienen. Bei solchen Systemen müssen die Abbildungseigenschaften so sein, dass ein mittels einer Lichtquelle erzeugtes Bild erhalten bleibt, was bei einer Bildpunkt-Lichtquelle, beispielsweise einer pixelierten Anzeige, bedeutet, dass einem Bildpunkt zugeordnete Strahlen, die den Wellenleiter an einem Auskoppelelements verlassen, weiterhin parallel zueinander sind und so bei Beobachtung mit einem menschlichen Auge ein gewünschtes Bild auf der Netzhaut erzeugen. Die vorliegende Anmeldung betrifft jedoch nicht solche Anordnungen, mit denen ein gemäß Daten veranschaulichtes Licht gleichsam projiziert wird, sondern die Beleuchtung von Objekten. Für viele Anwendungen ist es nötig, ein Objekt wie eine Probe zu beleuchten, z.B. um das Objekt zu untersuchen. US 2002/149924 A1 offenbart optischen Vorrichtungen mit einer Vielzahl von Flächenausgabemustern und einen Lichtejektor beschrieben, der das Licht durch eine Vielzahl von teilreflektierenden Grenzflächen in einen holografischen optischen Bauteil. An eine derartige Beleuchtung werden verschiedene Anforderungen hinsichtlich einer Leuchtverteilung des Beleuchtungslichts gestellt. Es ist eine Aufgabe, Möglichkeiten bereitzustellen, derartige Anforderungen zu erfüllen.

Diese Aufgabe wird von der Vorrichtung des Anspruchs 1 gelöst. Die abhängigen Ansprüche definieren bevorzugte Ausführungsbeispiele.

Erfindungsgemäß wird eine Vorrichtung zur Erzeugung einer Leuchtverteilung zum Beleuchten eines Objekts gemäß Anspruch 1 bereitgestellt.

Unter Licht wird hierbei elektromagnetische Strahlung verstanden. Es kann sich bei Licht beispielsweise um Licht im sichtbaren Wellenlängenbereich handeln, aber auch um Licht ganz oder teilweise im infraroten oder ultravioletten Spektralbereich. Auch können die Verfahren und Anwendungen Kombinationen verschiedener Lichtwellenlängenbereiche verwenden. Beispielsweise kann es vorteilhaft sein, wenn die Leuchtverteilung eine erste Lichtverteilung und eine zweite Leuchtverteilung umfasst, wobei die erste Leuchtverteilung für das menschliche Auge sichtbar ist und die zweite Leuchtverteilung nicht für das menschliche Auge sichtbar ist. Unter der Leuchtverteilung wird hierbei das von der Vorrichtung basierend auf dem eingekoppelten Licht bereitgestellte Lichtfeld verstanden.

Zumindest ein Teil der Replikationsbereiche kann eingerichtet sein, Licht aus dem Lichtwellenleiter auszukoppeln.

Die Replikationsbereiche sind eingerichtet, mindestens ein zugehöriges Eingangslichtbündel mit einem Eingangsstrahlprofil zu empfangen. Beispielsweise können einzelne

Replikationsbereiche eingerichtet sein, genau ein zugehöriges Eingangslichtbündel zu empfangen. In anderen Beispielen kann ein Replikationsbereich auch eingerichtet sein, mehrere Eingangslichtbündel zu empfangen. Diese können wiederum von einem einzelnen Replikationsbereich bereitgestellt werden oder aber auch von anderen Elementen, beispielsweise von einem Einkopplungselement und zwei verschiedenen Replikationsbereichen.

Mittels der Replikationsbereiche können gewünschte Leuchtverteilungen zum Beleuchten, z.B. von Objekten wie Proben, bereitgestellt werden, die verschiedene Anforderungen erfüllen können. Objekte können auch Raumbereiche wie Zimmer sein.

Eingangs- und Ausgangslichtbündel können hierbei verschiedene Formen aufweisen, sowohl räumlich scharf begrenzt als auch kontinuierlich geformt sein. Beispielsweise können sie rechteckige, quadratische, kreisförmige, hexagonale oder elliptische Querschnittsformen aufweisen, aber auch andere Formen und Kombinationen solcher Formen sind möglich.

Die oben erwähnten optischen Elemente sind erfindungsgemäß Volumenhologramme.

In Abhängigkeit von der konkreten Anwendung können optische Elemente für bestimmte Blickwinkel in bestimmten Wellenlängenbereichen, beispielsweise bei einem Blick entlang einer Flächennormalen des Lichtwellenleiters im sichtbaren Wellenlängenbereich des menschlichen Auges entweder transparent, teiltransparent oder intransparent sein.

Die Funktionalität der optischen Elemente, die in dem Lichtwellenleiter verbaut und/oder eingebettet sind, können mit zusätzlichen optischen Elementen, die außerhalb des Lichtwellenleiters oder auf diesem liegen wie Spiegeln, Prismen, Linsen, Mikrolinsen, Mikrolinsenarrays etc. ergänzt werden.

Manche der optischen Elemente können in dem Lichtwellenleiter gebildet werden. Dies wird manchmal auch als "Schreiben" bezeichnet. Beispielsweise können diffraktive Strukturen in oder auf dem Lichtwellenleiter mittels Laserschreiben erzeugt werden, aber auch andere Prozesse sind möglich.

Unter einer Kinoform wird hierbei ein diffraktives Element mit einem periodischen Höhenprofil verstanden. Das periodische Höhenprofil kann beispielsweise ein Sägezahnprofil sein. Replikationsbereiche können hierbei getrennt voneinander als diskrete Elemente ausgeführt sein. Replikationsbereiche können aber auch kontinuierlich ausgebildet sein, beispielsweise in Bereichen mit räumlich veränderlichen optischen Eigenschaften. Alternativ oder ergänzend können Replikationsbereiche auch überlappen, beispielsweise durch Mehrfachbelichtung eines bestimmten Ortes des Lichtwellenleiters, beispielsweise um mehrfachbelichtete Volumenhologramme zu erzeugen.

Die Vorrichtung kann zur Bereitstellung von kontinuierlichen und diskreten Leuchtverteilungen verwendet werden. Mit der Vorrichtung können beispielsweise Fixationsmarken, Justagemarken und/oder andere Muster als Leuchtverteilung bereitgestellt werden.

Dies kann es ermöglichen, kompakte Beleuchtungssysteme, beispielsweise für die Keratometrie, also die Vermessung der Hornhautform, bereitzustellen. Fixationsmarken und Muster können vorteilhaft in ophthalmischen Geräten verwendet werden.

Muster können sowohl im Ortsraum als auch im Frequenzraum der Leuchtverteilung realisiert werden.

Fixationsmarken können hierbei als Licht verstanden werden, welches dem Patienten in einer benötigten Entfernung als virtuelles Bild präsentiert werden. Derartige Fixationsmarken können auf bekannte Weise beispielsweise mit Hilfe einer von einer im sichtbaren Spektralbereich emittierenden Lichtquelle beleuchteten Maske generiert werden, die von einer klassischen Abbildungsoptik, beispielsweise einer Sammellinse, nach Unendlich abgebildet werden. Das virtuelle Bild wird dem Patienten innerhalb seines Gesichtsfeldes angeboten. Um die Marke scharf zu sehen, richtet der Patient sein Auge so aus, dass die Marke auf die Fovea Centralis, die Stelle des schärfsten Sehens, abgebildet wird und bringt sein Auge damit in die gewünschte Orientierung, so dass eine Messung durchgeführt werden kann.

Die beschriebenen Vorrichtungen können somit alternative Möglichkeiten zum Bereitstellen solcher Fixationsmarken bieten. Die erfindungsgemäß bereitgestellten Fixationsmarken können verbesserte Eigenschaften aufweisen, beispielsweise eine verbesserte Abbildungsqualität und/oder Lichteffizienz. Zusätzlich oder alternativ kann die Abmessung und/oder Komplexität der Vorrichtung gegenüber den bekannten Vorrichtungen zur Erzeugung von Fixationsmarken verbessert werden.

Die beschriebenen Vorrichtungen können beispielsweise auch verwendet werden, um Justagemarken und/oder Kalibrierungstargets für abbildende optische Geräte zur Verfügung zu stellen. Manche der beschriebenen Vorrichtungen erlauben eine vorteilhafte Beleuchtung einer mikroskopischen Probe, beispielsweise aus diskreten Richtungen, beispielsweise zur winkelvariablen Beleuchtung.

Häufig ist es wünschenswert, dass die Leuchtverteilung eine bestimmte Intensitätsverteilung aufweist. Hierfür kann es erforderlich sein, die Stärke der einzelnen Kopplungen, beispielsweise zwischen den jeweiligen Replikationselementen, räumlich zu variieren. Beispielsweise kann eine homogene Intensitätsverteilung der Leuchtverteilung gewünscht sein. Mit anderen Worten kann es beispielsweise gewünscht sein, dass alle in Richtung eines Objekts propagierenden kollimierten Bündel, die beispielsweise die Leuchtverteilung bereitstellen können, die gleiche Leistung transportieren. Hierbei kann jedoch die Anzahl von Replikationsbereichen, die das Licht durchläuft, bevor es in Richtung des Objekts umgelenkt wird unterschiedlich sein, beispielsweise weil einige Replikationsbereiche räumlich näher an dem mindestens einen Einkopplungselement liegen als andere. Hat die Vielzahl von Replikationsbereichen beispielsweise gleiche Abmessungen und ist z.B. rechteckig angeordnet, und wäre eine Auskopplungseffizienz entlang einer Richtung der jeweiligen Ausgangslichtbündel konstant bei 10 %, würden bei der ersten Interaktion zwischen der im Lichtwellenleiter propagierenden Strahlung und einem solchen Replikationsbereich 10 % des Lichts beispielsweise als Abgabelicht ausgekoppelt, während 90 % als Ausgangslichtbündel weiter zu einem weiteren Replikationsbereich weiterpropagieren würden und dort als Eingangslichtbündel empfangen würden. Bei einer zweiten Interaktion würden nur noch 9 % des Lichts als Abgabelicht ausgekoppelt, bei einer dritten Interaktion 8,1 % usw. Daher kann es vorteilhaft sein, die Kopplungseffizienz und/oder Auskopplungseffizienz der optischen Elemente, die für die Bereitstellung der Leuchtverteilung verwendet werden, zu variieren. Dies kann beispielsweise im Hinblick auf die gewünschte Leuchtverteilung und unter Berücksichtigung einer bereits durchlaufenden Anzahl von Kopplungen erfolgen. Bei kontinuierlich ausgeführten Replikationsbereichen kann beispielsweise eine Propagationslänge als ein Kriterium herangezogen werden.

Um das Wellenleitersystem vor Verschmutzung, mechanischen Beschädigungen oder der Schädigung durch Reinigungsmittel/Reinigungsprozesse zu schützen, kann die Vorrichtung mit weiteren Elementen, z.B. Planscheiben kombiniert werden. Es sind sowohl Varianten mit als auch ohne Luftspalt denkbar.

Der Lichtwellenleiter kann eingerichtet sein, das Licht mit einer ersten Modulation zu empfangen. Hierbei kann die Vorrichtung so eingerichtet sein, dass die Leuchtverteilung eine zweite Modulation aufweist, wobei die zweite Modulation eine größere Anzahl an Extremwerten als die erste Modulation aufweist.

Unter einer Modulation wird hierbei verstanden, dass die Intensität von Licht sich als Funktion einer Variable ändert, beispielsweise von dunkel zu hell oder von hell zu dunkel. Die Variable kann ein Ort sein, beschrieben durch eine oder mehrere Ortskoordinaten, beispielsweise wenn das Licht auf einem Schirm abgebildet wird. Hierbei kann die Variable auch auf die Größe des Schirms bzw. der Abmessung des Lichts oder der Leuchtverteilung normiert werden. Die Variable kann auch ein Winkel oder mehrere Winkel sein, beispielsweise bezüglich eines Mittelpunkts des Einkopplungselements oder eines Mittelpunkts des Objekts definiert, beispielsweise als ein Azimut- und/oder ein Polarwinkel. Dann kann die Modulation von Licht durch eine Anzahl von Extremwerten der Intensität des betreffenden Lichts beschrieben werden. Extremwerte können hierbei Maximal- oder Minimalwerte sein, beispielsweise die Zahl von Hell-Dunkel- oder Dunkel-Hell-Übergängen bezüglich der Variable.

Die zweite Modulation weist eine größere Anzahl an Extremwerten auf als die erste Modulation, wenn die Anzahl der Extremwerte, beispielsweise Maxima oder Minima der Intensität der Leuchtverteilung, über eine Variable größer als die Anzahl der Extremwerte der Intensitätsänderungen des Lichts über eine andere oder die Variable ist.

Die erste Modulation kann hierbei die Modulation des Lichts am Einkopplungselement sein.

Die erste und zweite Modulation können hierbei beispielsweise bezüglich eines Ortsraums oder in einem Winkelraum bestimmt werden. Als Ortsraum kann beispielsweise die Modulation bezüglich Ortskoordinaten in einer Ebene senkrecht zu der Ausbreitungsrichtung des jeweiligen Lichts, dessen Modulation bestimmt werden soll, gewählt werden. In anderen Beispielen kann die Modulation als Funktion eines Winkels bestimmt werden, beispielsweise wenn das Licht nicht kollimiert ist, beispielsweise wenn das Licht auf ein Objekt hin effektiv fokussiert ist.

Es kann hierbei zweckdienlich sein, passende Arten der Modulationsbestimmung in Abhängigkeit der Art des Lichts zu wählen. Beispielsweise kann eine winkelabhängige Modulation von kollimiertem Licht schwer zu bestimmen sein. In solchen Fällen kann eine Bestimmung der Modulation im Ortsraum, beispielsweise normiert auf den Strahldurchmesser oder die Leuchtverteilung, vorteilhaft sein. Eine Normierung kann zweckdienlich beispielsweise auf eine Fläche z.B. der Leuchtverteilung oder eine oder mehrere charakteristische Längen, beispielsweise einen Strahldurchmesser oder jeweilige Strahlhauptachsen, bezogen sein.

Bei fokussiertem Licht wiederum kann eine Bestimmung der Modulation im Winkelraum vorteilhaft sein.

Dies kann den Vorteil haben, dass mit der Vorrichtung komplexe Beleuchtungsverteilungen erzeugt werden können, ohne dass eine Lichtquellenanordnung, die das Licht an das Einkopplungselement bereitstellt, eine hohe Komplexität im Strahlprofil aufweisen muss.

Eine Lichtquellenanordnung, die beispielsweise eine einzelne LED mit einem Kollimator umfasst, weist beispielsweise als Funktion eines Winkels bezogen auf einen Verbindungsvektor zwischen der Lichtquellenanordnung und einem Einkopplungselement des mindestens einen Einkopplungselements eine Dunkel-Hell-Dunkel-Modulation auf. Die Anzahl der Extremwerte einer solchen Lichtquellenanordnung kann als ein Maximum oder als zwei Minima gezählt werden.

Wenn die Leuchtverteilung in einem nicht beanspruchten Beispiel ebenfalls einen Dunkel-Hell-Dunkel-Bereich aufweist, ist die Anzahl der Extremwerte der zweiten Modulation gleich der ersten Modulation, wenn die Anzahl der Extremwerte konsistent bestimmt wird, also Anzahl der Minima mit Anzahl der Minima oder Anzahl der Maxima mit Anzahl der Maxima verglichen wird. Dies ist für abbildende Systeme üblich.

Somit kann beispielsweise aus dem Licht einer einzelnen LED von der Vorrichtung eine komplexe Leuchtverteilung bereitgestellt werden. Eine solche komplexe Leuchtverteilung kann eine größere Anzahl an Extremwerten der Modulation aufweisen als die Anzahl der Extremwerte der Modulation der Lichtquellenanordnung.

Beispielsweise kann die Leuchtverteilung eine Dunkel-Hell-Dunkel-Hell-Dunkel-Modulation als die zweite Modulation aufweisen, also zwei Maxima bzw. drei Minima. In diesem Fall ist die Anzahl der Extremwerte der zweiten Modulation größer als die Anzahl der Extremwerte der ersten Modulation. Dies kann auch für komplexere Lichtquellenanordnungen entsprechend durchgeführt werden. Beispielsweise kann der Lichtwellenleiter eingerichtet sein, Licht von fünf diskreten Lichtquellen zu empfangen und eine Leuchtverteilung bereitzustellen, die mehr als fünf diskrete Lichtpunkte bildet, beispielsweise zehn Lichtpunkte. Aber auch andere Zahlen von Lichtpunkten größer fünf und andere Muster als Punktmuster sind möglich.

In einem anderen Beispiel kann eine Lichtquellenanordnung räumlich im Wesentlichen unmoduliertes Licht zur Einkopplung in den Lichtwellenleiter bereitstellen und die Leuchtverteilung moduliert sein. Im Wesentlichen bedeutet hierbei, dass eine Lichtquelle intrinsische - gegebenenfalls unerwünschte - Modulationen der Intensität aufweisen kann. Beispielsweise kann Laserstrahlung Transversalwellen, manchmal als transversalelektromagnetische Moden $\mathrm{TEM}_{xy}$ bezeichnet, aufweisen. Aber auch andere Intensitätsmodulationen einer Lichtquelle können vorliegen.

Bei manchen Ausführungsformen umfasst die Vorrichtung keinen räumlichen Lichtmodulator, der eingerichtet ist, in den Lichtwellenleiter einzukoppelndes Licht basierend auf Daten zu modulieren.

Beispiele für solche räumliche Lichtmodulatoren sind ein pixeliertes Display und ein Mikrospiegelaktuator (Englisch: digital micromirror device, DMD). Es geht hier also nicht darum, ein mittels einer derartigen Lichtmodulation erzeugtes Bild zu erzeugen.

Zumindest eine Teilmenge der Menge der Vielzahl von Replikationsbereichen kann eine Teilleuchtverteilung der Leuchtverteilung bereitstellen. Diese Teilleuchtverteilung kann eine effektive Fokussierung aufweisen.

Diese Teilleuchtverteilung kann eine effektive Defokussierung aufweisen.

Unter einer effektiven Fokussierung wird im Rahmen dieser Anmeldung verstanden, dass das Abgabelicht, welches den Lichtwellenleiter von einer gedachten Abgabefläche aus verlässt, auf eine gedachte Fokusfläche hin zuläuft, wobei die gedachte Fokusfläche kleiner als die Abgabefläche ist.

Es kann auch möglich sein, das die Teilleuchtverteilung oder eine zweite Teilleuchtverteilung eine effektive Defokussierung aufweist. Die gedachte Fokusfläche kann hierbei für einen virtuellen Strahlengang in virtuell fortgesetzter umgekehrter Lichtrichtung zusammenlaufen, wie dies beispielsweise von der Definition von negativen Brennweiten für Zerstreuungslinsen bekannt ist. Der reale Strahlengang kann für Licht, das auf eine gedachte Ebene, die in Strahlrichtung von der Abgabefläche entfernt ist, somit auseinanderlaufen.

Die optischen Elemente sind Volumenhologramme. Das mindestens eine Volumenhologramm kann gerade oder schräg innerhalb des Lichtwellenleiters angeordnet sein. Das Volumenhologramm kann mehrfachbelichtet sein.

Durch eine schräge Anordnung eines Volumenhologramms in dem Lichtwellenleiter können mögliche Lücken in der Leuchtverteilung reduziert werden.

Lücken in der Leuchtverteilung können beispielsweise auftreten, wenn von den Replikationsbereichen replizierte Lichtbündel Lücken in der Nähe des Lichtwellenleiters aufweisen. Beispielsweise können Replikationsbereiche räumlich voneinander beabstandet angeordnet sein, sodass die Leuchtverteilung, die beispielsweise durch ein Auskopplungselement bereitgestellt werden kann, nahe dem Lichtwellenleiter Lücken aufweisen kann. Je nach Abstand der Lücken und Entfernung zu einem beleuchteten Objekt können auch Lücken in der lateralen Ausleuchtung auf dem Objekt entstehen. Solche Lücken können in manchen Anwendungsfällen vorteilhaft sein, beispielsweise wenn Fixationsmarken bereitgestellt werden, die Lücken aufweisen sollen. In anderen Anwendungsfällen können solche Lücken unproblematisch sein, beispielsweise wenn die Leuchtverteilung eine Lücke von 1 mm am Objekt, beispielsweise einem menschlichen Auge, aufweist, das Objekt jedoch eine Pupille, beispielsweise eine Augenpupille des menschlichen Auges, von 3 mm aufweist.

In anderen Ausführungsbeispielen, beispielsweise bei der Probenbeleuchtung in der Mikroskopie, können solche Lücken jedoch unerwünscht sein. Bei für derartige Lücken empfindliche Applikationen kann es vorteilhaft sein, Lücken der Leuchtverteilung zu minimieren bzw. gänzlich zu verhindern.

In Ausführungsbeispielen ohne schräg angeordnete Volumenhologramme können die Lücken zwischen Lichtbündeln von diskreten Replikationsbereichen von folgenden Parametern abhängen: Der Form und dem Durchmesser des kollimierten Bündels, der Größe des Einkopplungselements, dem Propagationswinkel des Bündels nach Ablenkung durch das

Einkopplungselement, beispielsweise beschrieben durch einen Einkopplungswinkel zu einer Flächennormalen des Lichtwellenleiters im Bereich des Einkopplungselements $\alpha$, und der Dicke d des Lichtwellenleiters. Unter der Annahme eines vollständig von dem kollimierten Lichtbündel ausgeleuchteten quadratischen Einkopplungselements mit einer Kantenlänge b können Lücken zwischen den Lichtbündeln, welche die Leuchtverteilung bilden, durch Einhalten der Beziehung

$$b/2 > d * \tan(\alpha) \tag{1}$$

vermieden werden. Gleichzeitig muss $\alpha$ größer als ein Grenzwinkel der Totalreflexion des Lichtwellenleiters sein, um die nahezu verlustfreie Strahlungspropagation im Lichtwellenleiter zu gewährleisten.

Somit können durch eine entsprechende Auslegung der Parameter b, d und $\alpha$ für solche Systeme Lücken in der Leuchtverteilung reduziert oder vollständig vermieden werden.

Durch schräge Anordnung von Volumenhologrammen kann in manchen Ausführungsbeispielen eine erforderliche Stärke des Lichtwellenleiters, die erforderlich ist, um eine gewünschte Leuchtverteilung zu erzeugen und Lücken zu vermeiden, verringert werden.

Mit anderen Worten kann es bei schräg angeordneten Volumenhologrammen möglich sein, lückenreduzierte oder lückenfreie Leuchtverteilungen auch für Lichtwellenleiter bereitzustellen, die die Bedingung von Gleichung (1) nicht erfüllen, beispielsweise eine geringere Dicke d aufweisen.

Die Vorrichtung kann eine Lichtquellenanordnung umfassen. Die Lichtquellenanordnung kann eingerichtet sein, das Licht bereitzustellen. Die Lichtquellenanordnung kann mindestens eines der folgenden Elemente umfassen: zwei Lichtquellen, die eingerichtet sind, Licht in unterschiedlichen Richtungen und/oder in unterschiedlichen Wellenlängenbereichen und/oder an unterschiedliche Beleuchtungspositionen des mindestens einen Einkopplungselements bereitzustellen.

Hierdurch können komplexe Leuchtverteilungen, beispielsweise Superpositionen von Leuchtverteilungen bei verschiedenen Wellenlängen, erzeugt werden, ohne komplexe Lichtquellen zu benötigen.

Die Lichtquellenanordnung kann alternativ oder ergänzend weitere Elemente, wie beispielsweise Strahlteiler, Scanspiegel und/oder ein schaltbares Element, umfassen.

Die Vielzahl von Replikationsbereichen umfasst eine erste Gruppe von Replikationsbereichen die jeweils miteinander optisch gekoppelt sind. Die Replikationsbereiche der ersten Gruppe von Replikationsbereichen sind jeweils eingerichtet um: Mindestens ein erstes zugehöriges Ausgangsbündel der Vielzahl von Ausgangslichtbündeln an einen anderen Replikationsbereich der ersten Gruppe von Replikationsbereichen bereitzustellen und mindestens ein zweites zugehöriges Ausgangsbündel der Vielzahl von Ausgangslichtbündeln nicht an einen anderen Replikationsbereich der ersten Gruppe von Replikationsbereichen bereitzustellen, um eine Menge von Abgabebündel der ersten Gruppe von Replikationsbereichen zu erhalten.

Die Menge von Abgabebündeln kann eingerichtet sein, die Leuchtverteilung bereitzustellen. Die optische Kopplung kann eine Reihenstruktur aufweisen.

Die optische Kopplung kann eine Baumstruktur aufweisen.

Unter einer Baumstruktur der optischen Kopplung wird hierbei verstanden, dass die Replikationsbereiche der ersten Gruppe von Replikationsbereichen jeweils mindestens zwei der Vielzahl von zugehörigen Ausgangslichtbündeln an mindestens zwei Replikationsbereiche als jeweilige Eingangslichtbündel bereitstellen.

In einem Beispiel kann ein Replikationsbereich der ersten Gruppe von Replikationsbereichen drei Ausgangslichtbündel aufweisen. In einer ersten Variante des Beispiels können zwei Ausgangslichtbündel als Eingangslichtbündel bereitgestellt werden. So wird eine Baumstruktur bereitgestellt, die mit $2^n$, wobei n die Anzahl der Replikationsbereiche bezeichnet, verzweigt. Das dritte Ausgangslichtbündel kann aus dem Lichtwellenleiter ausgekoppelt werden.

In einer zweiten Variante des Beispiels können alle drei Ausgangslichtbündel als Eingangslichtbündel bereitgestellt werden, wodurch eine $3^n$ Baumstruktur gebildet wird. Das Licht kann von weiteren Replikationsbereichen und/oder separaten Auskopplungselementen oder Kombinationen davon an Zweigen der Baumstruktur ausgekoppelt werden.

In anderen Beispielen können andere Verzweigungen bereitgestellt werden, allgemein $y^n$, wobei y die Anzahl der Ausgangslichtbündel oder die Anzahl der Ausgangslichtbündel -1 ist.

Auch eine Kombination verschiedener optischer Kopplungen ist möglich, beispielsweise kann zunächst eine Baumstruktur optisch gekoppelt werden und innerhalb eines einzelnen Zweiges der Baumstruktur können dann optische Elemente wiederum in Reihenstruktur optisch gekoppelt sein.

Die Vielzahl von Replikationsbereichen umfasst eine zweite Gruppe von Replikationsbereichen die jeweils miteinander optisch gekoppelt sind. Eine Teilmenge der zweiten Gruppe von Replikationsbereichen ist eingerichtet die Menge von Abgabebündeln der ersten Gruppe von Replikationsbereichen als jeweilige Eingangslichtbündel zu empfangen.

Die optische Kopplung der ersten Gruppe von Replikationsbereichen und/oder der zweiten Gruppe von Replikationsbereichen kann eine Reihen-optische-Kopplung umfassen und/oder die optische Kopplung kann eine Baumstruktur umfassen. Auch in diesen Beispielen sind Kombinationen von Reihen- und Baumstrukturen möglich.

Wie zuvor beschrieben, kann zumindest ein Teil der Replikationsbereiche eingerichtet sein, Licht aus dem Lichtwellenleiter auszukoppeln. Eine weitere Möglichkeit der Lichtauskopplung, die alternativ oder ergänzend verwendet werden kann, wird nachfolgend beschrieben.

Die optischen Elemente können ferner umfassen: Mindestens ein Auskopplungselement, welches eingerichtet ist, Licht aus dem Lichtwellenleiter auszukoppeln.

Dieses mindestens eine Auskopplungselement kann ebenfalls als ein Replikationsbereich wirken. Wie zuvor beschrieben, kann auch ein Replikationsbereich oder mehrere Replikationsbereiche als Auskopplungselement wirken. Mit anderen Worten kann die Funktionalität von Replikationsbereichen und Auskopplungselementen zusammenfallen und/oder ergänzend wirken.

Auch eine Kombination verschiedener Auskopplungsarten ist möglich. Beispielsweise kann in einem ersten Bereich des Lichtwellenleiters Licht von einem oder mehreren Replikationsbereichen ausgekoppelt werden und in einem zweiten Bereich des Lichtwellenleiters Licht von einem oder mehreren Auskopplungselementen ausgekoppelt werden.

Ferner ist es möglich, dass mindestens ein Auskopplungselement Licht aus mindestens zwei verschiedenen Richtungen, beispielsweise von mindestens zwei Replikationsbereichen empfängt. Das Auskopplungselement kann dann ein oder mehrere Lichtbündel als das Abgabelicht oder einen Teil des Abgabelichts bereitstellen. Hierbei kann ein solches mindestens eines Auskopplungselement Licht mit einer jeweils gleichen Orientierung empfangen, beispielsweise Teile von Lichtbündeln, die sich in Totalreflektion in dem Lichtwellenleiter bewegen und von jeweiligen Replikationsbereichen mit gleicher Orientierung an das Auskopplungselement bereitgestellt werden. Das mindestens eine Auskopplungselement kann mehrere Eingangsbündel mit gleicher oder ähnlicher Orientierung empfangen und in eines oder mehrere Ausgangsbündel umwandeln, um zumindest einen Teil der Leuchtverteilung bereitzustellen. Hierbei können die mehreren Ausgangsbündel unterschiedliche Orientierungen und/oder Wellenfrontformen aufweisen.

Alternativ oder ergänzend kann ein Auskopplungselement zum Auskoppeln ohne Replikation eingerichtet sein. Mit anderen Worten kann ein solches Auskopplungselement nicht mit anderen Replikationsbereichen und/oder anderen Auskopplungselementen optisch gekoppelt sein. Dies kann beispielweise verwendet werden, um Randbereiche der Leuchtverteilung bereitzustellen.

Das mindestens eine Auskopplungselement und/oder das mindestens eine Einkopplungselement können ein oder mehrere weitere optische Elemente umfassen, beispielsweise eine Linse, ein Prisma, ein Oberflächengitter, einen Polarisationsfilter.

Diese optischen Elemente können verwendet werden, um die Qualität der Leuchtverteilung weiter zu verbessern und/oder die Freiheitgrade für die Gestaltung Leuchtverteilung zu erhöhen.

Die Leuchtverteilung kann so eingerichtet sein, dass eine Vielzahl von Strahlen von unterschiedlichen Bereichen des Lichtwellenleiters derart abgegeben werden, so dass das abgegebene Licht effektiv fokussiert und/oder effektiv defokussiert ist.

Die Vielzahl von Strahlen können kollimiert sein und/oder in diskreten Winkelbereichen aus dem Lichtleiter abgegeben werden.

Derartige Leuchtverteilungen können für einige Anwendungsfälle vorteilhaft sein, beispielsweise in der Keratometrie und Mikroskopie.

Hierdurch können unterschiedliche Stellen eines Objekts, beispielsweise im Falle der Keratometrie des menschlichen Auges, unter verschiedenen Winkeln von kollimierten Strahlen beleuchtet werden.

Das mindestens eine Auskopplungselement kann mindestens ein weiteres optisches Element umfassen. Dieses kann eingerichtet sein, um ein Muster des ausgekoppelten Lichts zu erzeugen.

Ein solches Muster kann beispielsweise ein Strich und/oder Rechteck und/oder Wabe und/oder eine Kreuz-Form sein. Mit anderen Worten können die optischen Elemente, die Bestandteil des Lichtwellenleiters sind, durch weitere optische Elemente ergänzt werden. Beispielsweise kann in einem Ausführungsbeispiel ohne weitere optische Elemente Licht von einem Replikationsbereich bereitgestellt werden, um einen Teil der Leuchtverteilung bereitzustellen. In einem anderen Ausführungsbeispiel kann die Vorrichtung mindestens ein weiteres optisches Element umfassen, beispielsweise eine Linse. In diesem Ausführungsbeispiel kann dann das Licht, welches von dem Replikationsbereich bereitgestellt wird, aus dem Lichtwellenleiter kommend die Linse durchlaufen und nach Durchlaufen des mindestens einen weiteren optischen Elements einen Beitrag zu der Leuchtverteilung der Vorrichtung leisten. Hierbei kann das mindestens eine weitere optische Element einstückig mit dem Lichtwellenleiter geformt sein, es kann aber auch unabhängig davon ausgeführt sein, beispielsweise auf den Lichtwellenleiter angebracht sein oder mit einer Halterung mit einem Abstand vom Lichtwellenleiter angeordnet sein. Ebenfalls kann eine Funktionsintegration der weiteren optischen Elemente durchgeführt werden. Beispielsweise kann ein weiteres optisches Element Bestandteil eines Auskopplungselements sein, beispielsweise indem das Auskopplungselement eine gekrümmte Oberfläche aufweist und hierdurch zusätzlich als Sammellinse wirkt.

Beispielsweise kann das Einkopplungselement als ein diffraktives Element, beispielsweise ein

Oberflächengitter, ausgeführt sein. Der erste Replikationsbereich ist als Volumenhologramm ausgeführt.

Das mindestens eine weitere optische Element kann ein schaltbares diffraktives Element sein. Durch solche schaltbaren Elemente kann die von der Vorrichtung bereitgestellte Leuchtverteilung modifiziert werden.

Das mindestens eine Einkopplungselement kann eingerichtet sein, ein Einkoppeln basierend auf einer Charakteristik des Lichts vorzunehmen. Die Replikationsbereiche können eingerichtet sein, für mindestens zwei verschiedene Charakteristiken des Lichts mindestens zwei verschiedene zugehörige Leuchtverteilungen zu erzeugen.

Charakteristiken des Lichts können beispielsweise Wellenlänge, Polarisation und Einkopplungswinkel, Position der Einkopplung, aber auch Kombinationen davon, sein. Hierdurch können ebenfalls verschiedene Leuchtverteilungen von einer Vorrichtung bereitgestellt werden. Durch Variation der Lichtcharakteristik kann die Leuchtverteilung der Vorrichtung dann gesteuert werden, ohne dass die Vorrichtung selbst aktive Komponenten umfassen muss. So kann beispielsweise eine Steuerung die Charakteristik des Lichts aus der Lichtquellenanordnung modifizieren.

In wieder anderen Ausführungsbeispielen können auch verschiedene Steuerungsmöglichkeiten gekoppelt werden, beispielsweise können optische Elemente, die im Lichtwellenleiter angeordnet sind, steuerbar sein und gleichzeitig eine Charakteristik des Lichts variiert werden. Hierdurch können Leuchtverteilungen gewechselt werden und zahlreiche Varianten von gewünschten Beleuchtungsverteilungen mit verhältnismäßig wenig komplexen Elementen erzielt werden.

Die Vorrichtung kann eingerichtet sein, eine Leuchtverteilung zum winkelvariablen Beleuchten eines von der Vorrichtung entfernten Objekts bereitzustellen, wobei das Objekt einen kleineren Durchmesser als der Lichtwellenleiter aufweist.

Unter einer winkelvariablen Beleuchtung wird hierbei ein einstellbares Beleuchten aus verschiedenen Winkeln und verschiedenen Richtungen verstanden.

Vorrichtungen zum winkelvariablen Beleuchten sind beispielsweise in den Anmeldungen DE 10 2014 101 219 A1, DE 10 2016 116 311 A1 und DE 10 2014 112 242 A1 beschrieben.

Um zu bestimmen, ob das Objekt einen kleineren Durchmesser als der Lichtwellenleiter aufweist, können jeweilige Durchmesser von Objekt und Lichtwellenleiter in Richtung der Leuchtverteilung berücksichtigt werden. Beispielsweise kann der Lichtwellenleiter in einer Richtung, in der keine Leuchtverteilung bereitgestellt wird, auch eine geringere Abmessung aufweisen als der Durchmesser des Objekts. Beispielsweise kann der Lichtwellenleiter wenige Millimeter dünn ausgeführt sein, aber eine laterale Größe von einigen Zentimetern aufweisen und ein Objekt von 5 mm Durchmesser beleuchten.

Die Vorrichtung kann eingerichtet sein, die Leuchtverteilung für das Objekt bereitzustellen, wenn das Objekt unter einem Winkel zu einer Flächennormale des Lichtwellenleiters angeordnet ist.

Dies kann den Vorteil haben, dass Vorrichtungen einen größeren Designfreiheitsgrad für eine gewünschte Leuchtverteilung aufweisen. Dies kann beispielsweise Funktionale und/oder ästhetische Vorteile bieten, beispielsweise wenn die Vorrichtung in einer Brille eingebaut ist und verwendet wird um ein Augenimplantat im Auge eines Benutzers mit Energie zu versorgen. In solchen Beispielen, aber nicht darauf beschränkt, kann es vorteilhaft sein, wenn ein Winkel zu der Flächennormale des Lichtwellenleiters gewählt werden kann. Dies kann beispielsweise die Ästhetik einer solchen Brille verbessern.

Gemäß einem Ausführungsbeispiel wird ein Lichtwellenleitersystem mit einer Vielzahl von Vorrichtungen bereitgestellt. Die Vielzahl von Vorrichtungen ist jeweils gemäß einem der vorherigen Ausführungsbeispiele ausgeführt, wobei die Vielzahl von Vorrichtungen einen gemeinsamen Lichtwellenleiter mit einer Auskopplungsfläche aufweist. Der gemeinsame Lichtwellenleiter kann mindestens eine Aussparung mit einer Aussparungsfläche in der Auskopplungsfläche aufweisen. Die Vielzahl von Vorrichtungen kann so angeordnet sein, dass die Leuchtverteilungen der Vielzahl von Vorrichtungen von mindestens 80 % der Auskopplungsfläche ohne die Aussparungsfläche ausgehen.

Dies kann den Vorteil haben, dass es möglich sein kann das Lichtwellenleitersystem zu einer Beleuchtung zu verwenden, wobei die von dem Lichtwellenleitersystem erzeugte Leuchtverteilung von einem Detektionssystem beobachtet werden kann. Durch die Aussparung kann ein hoher Freiheitsgrad der Leuchtverteilung erreicht werden, ohne dass der Lichtwellenleiter das Detektionssystem negativ beeinflusst. Dies kann insbesondere vorteilhaft sein, wenn das Lichtwellenleitersystem als alternatives Beleuchtungssystem für eine Messanordnung verwendet werden soll und die Messanordnung nicht neu qualifiziert werden soll oder das Lichtwellenleitersystem in eine bestehende Messanordnung integriert werden soll, beispielsweise nachgerüstet werden soll. Ein solches Lichtwellenleitersystem kann ebenfalls vorteilhaft sein, wenn Antwortlicht, welches in Reaktion auf die Leuchtverteilung, beispielsweise von einem Objekt ausgeht, nicht durch Wechselwirkung mit einem Lichtwellenleiter modifiziert werden soll. Durch eine oder mehrere Aussparungen können in solchen Fällen unerwünschte Effekte wie Brechung, Reflexion, Kohärenzverlust etc. vermieden werden.

In der Keratometrie wird beispielweise eine vignettierungsfreie, telezentrische Detektion des von der Hornhaut zurückreflektierten Lichtes benötigt. Dies kann mittels einer Aussparung zwischen dem Auge und einem Detektor gewährleistet werden.

Der Lichtwellenleiter kann eine erste und eine zweite Seite aufweisen. Hierbei kann die Leuchtverteilung eine erste Leuchtverteilung auf der ersten und eine zweite Leuchtverteilung auf der zweiten Seite des Lichtwellenleiters umfassen.

Nachfolgend werden Beispiele der zuvor beschriebenen Vorrichtung im Hinblick auf verschiedene konkrete Anwendungsmöglichkeiten beschrieben. Die beschriebenen Anwendungsbeispiele sind als Beispiele zu verstehen. Die nachfolgend erörterten Aspekte der Anwendungsbeispiele können daher auch losgelöst von den konkreten Anwendungsmöglichkeiten verwendet werden, sei es allgemein bei erfindungsgemäßen Vorrichtungen oder in anderen der beschriebenen Anwendungsmöglichkeiten.

Gemäß einem Anwendungsbeispiel wird ein Keratometer zum Vermessen der Hornhaut des menschlichen Auges bereitgestellt.

Die Keratometrie ist ein verbreitetes Verfahren der Ophthalmologie und widmet sich der Vermessung der Form von Hornhaut des menschlichen Auges. Die Bedeutung und grundsätzliche Funktionsweise dieses Messverfahrens wird beispielsweise in der DE 10 2011 102 355 A1 erläutert. Aus dem Stand der Technik sind verschiedene Anordnungen bekannt, beispielsweise sogenannte Littmann-Keratometer, die kollimierte Strahlbündel aus unterschiedlichen Richtungen auf die Hornhaut des Auges auswerfen und das reflektierte Licht auswerten. Hierbei hängt die Qualität der Auswertung von der Anzahl von kollimierten Bündeln ab. Häufig ist das Erzeugen einer hinreichend hohen Anzahl von kollimierten Lichtbündeln mit großem Aufwand verbunden und begrenzt die mögliche Qualität des Verfahrens. Placido-Scheiben-Keratometer basieren auf planaren oder gewölbten Scheiben mit mehreren konzentrischen, selbst leuchtenden Ringen. Die Reflexionen der Ringe an der Hornhaut können mit einem Kamerasensor abgebildet werden und ausgewertet werden. Aus der Deformation der Ringe kann die Topographie der Hornhaut ermittelt werden.

Aus der DE 10 2011 102 355 A1 ist eine Kombination von Littmann-Keratometern und Placido-Scheiben-Keratometern bekannt. Mithilfe von einer einzelnen oder weniger Strahlungsquellen wird hierbei ein Array von mehreren kollimierten Bündeln erzeugt, die aus unterschiedlichen Richtungen zur Augenpupille hin propagieren. Durch die Verwendung kollimierter Bündel ist das Prinzip robuster gegenüber axialen Ablagen des Auges, während gleichzeitig durch die große Anzahl an kollimierten Bündeln eine große Robustheit gegenüber lokalen Hornhautdefekten erreicht werden kann. Zusätzlich zeichnet sich das Verfahren durch eine hohe Lichteffizienz aus. Das optische Element der DE 10 2011 102 355 A1 verwendet eine Kombination aus reflektiven und refraktiven Flächen und besitzt eine komplexe Grundgeometrie, die zu großem Aufwand bei der Fertigung des Elements führen kann. Der Durchmesser der kollimierten Bündel ist durch die Größe der Freiformfacetten des verwendeten optischen Elements limitiert. Für eine hinreichende Toleranz der Vorrichtung gegenüber Bewegung des Auges, ist es erforderlich, dass die einzelnen Bündel einen vom Einstrahlwinkel abhängigen Mindestdurchmesser haben. Bei einer vorgegebenen Facettenanzahl ergibt sich hierdurch ein großer Abstand des optischen Elements zum Auge des Patienten und damit verbunden auch ein großer Durchmesser des optischen Elements. Im Zentrum des Elements nach DE 10 2011 102 355 A1 muss eine Öffnung vorgesehen sein, die eine vignettierungsfreie telezentrische Detektion des von der Hornhaut zurückreflektierten Lichtes ermöglicht. Im Bereich dieser Detektion können keine Beleuchtungsbündel eingestrahlt werden. Um die hierdurch entstehende Messlücke im zentralen Hornhautbereich zu minimieren, muss das Element möglichst weit vom Auge entfernt stehen, was zu größeren Scheibendurchmessern führt. Die nachführend erläuterten Ausführungsbeispiele ermöglichen es, die verschiedenen Keratometer mittels der beschriebenen Vorrichtung zu verbessern.

Für eine hohe Messgenauigkeit eines Keratometer-Gesamtsystems kann es vorteilhaft sein, eine Steigung der auf das Auge fallenden Bündels sehr genau, beispielsweise auf 1/500 des jeweiligen Ablenkwinkels gegenüber einer Normalen des Lichtwellenleiters, zu kennen. Die Anforderungen an die Genauigkeit der Hornhautvermessung können sich außerhalb des Pupillenbereichs des Auges reduzieren. Es kann vorteilhaft sein, zu berücksichtigen, dass die Geometrie des Lichtwellenleiters, das Ablenkverhalten der verschiedenen optischen Elemente sowie die Wellenlänge, Position und Orientierung der Lichtquelle von externen Parametern, beispielsweise der Temperatur, abhängig sein können. Die sich ändernde Wellenlänge der verwendeten Strahlung kann wiederum Einfluss auf das Verhalten der optischen Elemente, beispielsweise die Kopplung, haben. Auch weitere Faktoren, beispielsweise in den Lichtwellenleiter induzierten mechanischen Spannungen oder die Schwerkraft können die Orientierung der bereitgestellten Strahlenbündel beeinflussen. Um die Auswirkungen aller dieser Effekte auf das Messergebnis zu minimieren und um möglichst große Fertigungs- und Montagetoleranzen zu ermöglichen, kann eine Keratometer mit Lichtwellenleiter bei unterschiedlichen Temperaturen, Luftfeuchten und Betriebsbedingungen der Lichtquellen charakterisiert werden und dies bei einer Auswertung des Berücksichtigt werden. So kann durch Auswertung der während der Messung tatsächlich vorhandenen Parameter mit Hilfe zusätzlicher Sensoren, beispielsweise Temperatur- und/oder Feuchtesensor und durch Rückgriff auf gewonnene Kalibrierungskurven und/oder analytische oder numerische Modelle des Systemverhaltens eine hohe Messgenauigkeit gewährleistet werden. Solche Vorgehensweisen können bei den beschriebenen Keratometern Anwendung finden.

Das Keratometer gemäß einem Anwendungsbeispiel umfasst: eine Vorrichtung gemäß der vorherigen Ausführungsbeispiele, wobei die Vorrichtung eingerichtet ist, eine Leuchtverteilung umfassend eine Vielzahl von kollimierten Strahlenbündeln mit jeweils definierten Abgabebereichen auf dem Lichtwellenleiter an ein menschliches Auge auf einer ersten Seite des Lichtwellenleiters bereitzustellen.

Das Keratometer kann weiter eine Detektionsvorrichtung, die eingerichtet ist, von dem menschlichen Auge reflektiertes Licht der kollimierten Strahlenbündel zu empfangen, umfassen. Durch die hohe spektrale und angulare Selektivität der Vorrichtung kann der Lichtwellenleiter trotz hoher Effizienz bei der Bereitstellung der Beleuchtungsverteilung eine sehr gute Transparenz aufweisen. Dies kann für alle hier beschriebenen Ausführungsbeispiele gelten. Bei der Anwendung solcher Vorrichtungen in der Keratometrie kann das Auge des Patienten somit beispielsweise während der Messung beobachtet werden und es kann erleichtert werden, mit dem Patienten - inklusive Blickkontakt - zu kommunizieren. Auch können Messsysteme, beispielsweise Keratometer, aber auch andere Messsysteme, möglich sein, bei denen der Patient während der Messung die Umgebung weiterhin beobachten kann und bei denen reale Umgebung und virtuelle Leuchtverteilungen verschmelzen.

Das Keratometer oder andere Vorrichtungen können als kopfgetragene, brillenähnliche Messgeräte ausgeführt werden, die während normaler Alltagstätigkeiten des Patienten Messungen durchführen oder das Auge des Patienten durch Projektion von Mustern bewusst stimuliert.

Solche Vorrichtungen können auch in anderen Anwendungsbereichen eingesetzt werden, beispielsweise um ausgewählte Regionen des Patientenauges zur Therapie zu bestrahlen, beispielsweise mit Infrarotstrahlung.

Die Detektionsvorrichtung kann auf einer zweiten Seite des Lichtwellenleiters angeordnet sein und eingerichtet sein, das reflektierte Licht entlang eines Strahlengangs durch die mindestens eine Aussparung zu empfangen.

Der Lichtwellenleiter kann eingerichtet sein, eine Leuchtverteilung mit einer konzentrischen Ringstruktur bereitzustellen. Hierbei können die konzentrischen Ringstrukturen durchgehende Ringe sein. Es können aber auch Leuchtverteilungen mit Ringstrukturen bereitgestellt werden, die diskrete Richtungen und/oder dazwischenliegende Lücken aufweisen. Auch andere Formen, beispielsweise Ellipsen, sind möglich.

Die konzentrische Ringstruktur kann dabei eine Leuchtverteilung ähnlich einer Placidoscheibe aufweisen.

Dies kann den Vorteil haben, dass bekannte Auswertungsverfahren verwendet werden können und zugleich die Qualität der Messungen verbessert werden kann und/oder die Komplexität des Keratometers verringert werden kann.

Die Baumstruktur der Replikationsbereiche kann entlang einer radialen Richtung angeordnet sein.

Dies kann den Vorteil haben, dass die Leuchtverteilung homogene Beleuchtungseigenschaften in einer poloidaler Richtung, beispielsweise bei der konzentrischen Ringstruktur, aufweisen kann. Hierdurch können insbesondere Inhomogenitäten der Beleuchtungsintensität in poloidaler Richtung vermieden werden.

Ein Keratometer kann zwei Lichtquellen als die Lichtquelle umfassen. Diese zwei Lichtquellen können eingerichtet sein, Licht in unterschiedlichen Richtungen und/oder in unterschiedlichen Wellenlängenbereichen und/oder an unterschiedliche Beleuchtungspositionen des mindestens einen Einkopplungselements bereitzustellen.

Hierbei kann eine erste Lichtquelle der zwei Lichtquellen Licht im Infrarot abgeben und eine zweite Lichtquelle der zwei Lichtquellen Licht im sichtbaren Bereich abgeben. Das Auskopplungselement kann eingerichtet sein, das Licht der zweiten Lichtquelle als Fixationsmarken bereitzustellen.

Das Infrarotlicht kann in beispielsweise von einer LED und/oder einer Laserdiode und/oder einer Superlumineszenzdiode bereitgestellt werden. Das Infrarotlicht kann beispielsweise bei 825 nm oder 1064 nm ein Intensitätsmaximum aufweisen.

Fixationsmarken können Muster im sichtbaren Spektralbereich sein, die an mehreren Positionen von dem Lichtwellenleiter ausgehen. Fixationsmarken können als geformte und/oder kollimierte Strahlen bereitgestellt werden. Die Strahlen können unterschiedliche Propagationsrichtungen aufweisen oder parallel zueinander verlaufen. Auch Kombinationen sind möglich. Das Muster kann beispielsweise eine Kreuzform aufweisen, aber auch andere Formen wie einzelne Punkte oder ein einzelner Punkt im Unendlichen, Punktmuster, leere oder gefüllte Kreise, leere oder gefüllte Quadrate etc. sind möglich.

Beispielsweise kann eine Kreuzform aus fünf Punkten in der Leuchtverteilung erzielt werden. Jeder Punkt kann hierbei durch ein kollimiertes, auf das Auge einfallendes, Strahlenbündel erzeugt werden. Dadurch, dass die fünf kollimierten Bündel aus unterschiedlichen Richtungen auf das Auge einfallen, werden sie von der Augenlinse an unterschiedlichen Stellen der Netzhaut fokussiert und erzeugen dort fünf Bildpunkte.

Die Strahlen können von virtuellen Punkten vor oder hinter dem Auge des Patienten/Nutzers ausgehen bzw. auf diese konvergieren. Dies kann als Fixationsmarken im Endlichen bezeichnet werden und kann beispielsweise bei fehlsichtigen Patienten verwendet werden, oder um ein Nahblickziel darzustellen.

In manchen Anwendungen können komplexe Muster aus einem einzelnen kollimierten Eingangsbündel und-/oder einem einzelnen kollimierten Bündel innerhalb des Wellenleiters bereitgestellt werden.

Das Auskopplungselement kann hierbei beispielsweise als ein Volumenhologramm ausgebildet sein, um das Muster in der Leuchtverteilung bereitzustellen, aber auch eine Kombination anderer optischer Elemente zur Erzeugung eines Musters sind möglich.

Der Lichtwellenleiter kann eine Antwortlichteinkopplungsvorrichtung umfassen. Diese kann eingerichtet sein, das reflektierte Licht in den Lichtwellenleiter einzukoppeln und aus dem Lichtwellenleiter an die Auswerteeinrichtung weiterzuleiten.

Hierbei kann die Antwortlichteinkopplungsvorrichtung basierend auf einer Modifikation eines Strahlprofiles der Leuchtverteilung aufgrund der Reflektion am Auge ausgebildet sein.

Eine solche Antwortlichteinkopplungsvorrichtung kann den Vorteil haben, dass der optische Aufbau eines Detektionsstrahlengangs vereinfacht werden kann. Gleichzeitig können hierdurch kompaktere Keratometer bereitgestellt werden. Mit anderen Worten kann die Flexibilität der Vorrichtung zum Erzeugen einer Leuchtverteilung umgekehrt ebenfalls verwendet werden, um Licht einzukoppeln und an einen oder mehrere Detektoren weiterzuleiten. Hierbei kann der Lichtweg umgekehrt werden.

Solche Vorrichtungen können den Vorteil haben, dass der herkömmlicherweise von der Detektionsvorrichtung blockierte Raumwinkel anders genutzt werden kann. Beispielsweise kann hierdurch der zentrale Blick auf das Auge des Patienten möglich werden. Mit anderen Worten kann eine herkömmlich als sperriger optischer Aufbau erscheinende Vorrichtung erfindungsgemäß wie ein Fensterglas wirken. Dies kann die Untersuchung angenehmer machen. Auch kopfgetragene Systeme, die den Blick auf die Umgebung zulassen sind möglich. Die Antwortlichteinkopplungsvorrichtung kann Bestandteil des Lichtwellenleiters sein. Sie kann aber auch getrennt von dem Lichtwellenleiter ausgeführt sein, beispielsweise mit dem Wellenleiter verbunden sein, beispielsweise mit dem Lichtwellenleiter verklebt sein.

Die Antwortlichteinkopplungsvorrichtung kann ausgebildet sein, eine typische Verkrümmung der Wellenfront zu berücksichtigen. Diese Verkrümmung kann beispielsweise vorausberechnet werden und eine entsprechende Kompensationsfunktion in die zugeordneten Einkopplungselemente integriert werden. Hierfür kann ein Beobachtungsstrahlengang, der den Lichtweg innerhalb des Lichtwellenleiters berücksichtigt, telezentrisch aufgebaut sein.

Zum Beispiel können Vorrichtungen zur Projektion von Fixationsmarken und Mustern in ophthalmischen Geräten bereitgestellt werden.

Gemäß einem Anwendungsbeispiel wird eine Projektionsvorrichtung für ophthalmische Geräten bereitgestellt. Die Projektionsvorrichtung umfasst eine Vorrichtung gemäß einer der vorherigen Ausführungsbeispiele, wobei die Projektionsvorrichtung eine vielfach modulierbare Lichtquelle umfassen kann, die eingerichtet sein kann, das Licht an die Vorrichtung bereitzustellen. Die Vorrichtung kann eingerichtet sein, die Leuchtverteilung so bereitzustellen, dass für mindestens eine Modulation der vielfach modulierbaren Lichtquelle mindestens zwei modulierte Leuchtverteilungen bereitgestellt werden, wobei die mindestens zwei modulierten Leuchtverteilungen bezüglich Richtung und/oder Ort moduliert sind.

Die mindestens zwei modulierten Leuchtverteilungen können beispielsweise Marken und/oder Landoltringe und/oder Buchstaben und/oder Streifenmuster sein.

Beispielsweise kann die vielfach modulierbare Lichtquelle drei Modulationen A, B und C aufweisen. Hierbei kann für Modulation A von der Vorrichtung eine Leuchtverteilung bereitgestellt werden, die eine Superposition von vier modulierten Leuchtverteilungen ist. Für Modulation B kann die Vorrichtung eine Leuchtverteilung, die nicht moduliert ist, bereitstellen. Für Modulation C kann die Vorrichtung eine Leuchtverteilung, die eine Superposition von zwei weiteren modulierten Leuchtverteilungen ist, bereitstellen.

Die mindestens zwei modulierten Leuchtverteilungen können bezüglich Richtung und/oder Ort so moduliert sein, dass sie sich teilweise entsprechen und teilweise unterscheiden. Beispielsweise kann eine erste modulierte Leuchtverteilung ein zentrales Kreuz und einen peripheren Ring aufweisen. Eine zweite modulierte Leuchtverteilung kann das gleiche zentrale Kreuz aber mehrere periphere Ringe, die teilweise unterbrochen sein können, aufweisen. Gemäß einem weiteren Anwendungsbeispiel kann eine winkelvariable Probenbeleuchtung im Mikroskop bereitgestellt werden.

Die zuvor beschriebenen Vorrichtungen können als Probenbeleuchtungsvorrichtungen ausgeführt werden, beispielsweise als eine Ergänzung zu einem bestehenden Mikroskop. Gemäß einem Anwendungsbeispiel wird ein Mikroskop mit einem Strahlengang und einer Probenbeleuchtungsvorrichtung bereitgestellt. Die Probenbeleuchtungsvorrichtung umfasst dabei eine Vorrichtung gemäß der vorherigen Ausführungsbeispiele. Der Lichtwellenleiter kann eingerichtet sein, dass, wenn er in dem Strahlengang angeordnet ist, mit der Leuchtverteilung ein Muster auf einer Probe in dem Mikroskop erzeugt.

Das Muster kann ein schaltbares Muster sein.

Das Muster kann ein winkelvariables Muster sein. Das Muster kann winkelvariabel schaltbar sein. Mit anderen Worten können für jeden Replikationsbereich oder für einige Replikationsbereiche die Intensitätsverhältnisse der jeweiligen Ausgangslichtbündel der Vielzahl von Ausgangslichtbündeln variiert werden. Dies kann, wie zuvor beschrieben, durch die Replikationsbereiche selbst erreicht werden oder durch ein oder mehrere Auskopplungselemente, oder durch Zusammenwirken von mindestens einem Auskopplungselement mit den Replikationsbereichen.

Gemäß einem weiteren Anwendungsbeispiel werden Kalibriermarken für optische Geräte bereitgestellt.

Gemäß einem Anwendungsbeispiel wird eine Kalibrierungsvorrichtung für ein optisches Gerät, umfassend mindestens eine Vorrichtung gemäß vorheriger Ausführungsbeispiele, bereitgestellt. Die Leuchtverteilung kann eingerichtet sein, ein Testlichtfeld für das optische Gerät bereitzustellen.

Das Testlichtfeld kann auf Zoom- und/oder Fokusebenenstellungen und/oder eine Installationsposition der Kalibrierungsvorrichtung des optischen Gerätes abgestimmt sein.

Die Installationsposition kann eine Position innerhalb von optischen Elementen des optischen Geräts und in einem Strahlengang des optischen Geräts sein.

Gemäß einem Anwendungsbeispiel wird ein Planglas, Filterglas oder Schutzglas für ein Objektiv bereitgestellt. Dieses kann eine Kalibrierungsvorrichtung gemäß vorheriger Anwendungsbeispiele umfassen.

Gemäß einem weiteren Anwendungsbeispiel wird eine Flächenleuchte bereitgestellt. Diese umfasst eine Vorrichtung gemäß vorheriger Ausführungsbeispiele. Hierbei kann der Lichtwellenleiter in einer Richtung eine Dimension von weniger als 50 μm aufweisen.

Gemäß einem Anwendungsbeispiel wird ein Fenster bereitgestellt. Dieses umfasst:
ein Fensterglas, umfassend eine Vorrichtung gemäß vorheriger Ausführungsbeispiele und einen Fensterrahmen, umfassend eine Lichtquelle. Die Lichtquelle kann eingerichtet sein,

Infrarotlicht bereitzustellen. Die Lichtquelle kann ferner eingerichtet sein, das Licht an das mindestens eine Einkopplungselement bereitzustellen. Die Leuchtverteilung kann eingerichtet sein, das Infrarotlicht als eine Wärmequelle auf mindestens einer Seite des Fensterglases bereitzustellen.

In manchen Anwendungsbeispielen ist ein Fenster an einer Außenwand eines Zimmers angebracht. In diesen Fällen kann das Infrarotlicht auf einer Innenseite des Fensters bereitgestellt werden, beispielsweise um das Zimmer mit Wärme zu versorgen, wobei die Wärme als Strahlungswärme von dem Infrarotlicht bereitgestellt werden kann.

In einem anderen Anwendungsbeispiel ist das Fenster an einer Außenwand zwischen einem Raum und einem Wintergarten angebracht. In diesem Fall kann die Leuchtverteilung das Infrarotlicht auf beiden Seiten des Fensters bereitstellen, um sowohl Wintergarten als auch das Zimmer zu heizen.

Das Infrarotlicht kann in einem Spektralbereich von 1 bis 10 μm ein Intensitätsmaximum aufweisen.

Die Erfindung wird nachfolgend unter Bezugnahme auf die beigefügte Zeichnung anhand von Ausführungsbeispielen detailliert erläutert:

Fig. 1A zeigt eine Vorrichtung zur Erzeugung einer Leuchtverteilung gemäß verschiedener Ausführungsbeispiele.

Fig. 1B zeigt eine Vorrichtung entsprechend Fig. 1A gemäß einem anderen Ausführungsbeispiel.

Fig. 2 zeigt eine Frontalansicht einer Vorrichtung entsprechend Fig. 1A und/oder Fig. 1B.

Fig. 3 zeigt ein Beispiel einer Vorrichtung mit einer Baumstruktur.

Fig. 4 zeigt ein weiteres Ausführungsbeispiel der Fig. 1A und der Fig. 1B.

Fig. 5 zeigt eine weitere Variante der Vorrichtung der Fig. 4.

Fig. 6 zeigt Vorrichtungen gemäß verschiedener nicht erfinderischer Ausführungsbeispiele, die mehrkanalig und/oder schaltbar sind.

Fig. 7A zeigt eine Seitenansicht eines Keratometers.

Fig. 7B zeigt eine Frontalansicht des Keratometers der Fig. 7A.

Fig. 8 zeigt ein weiteres Ausführungsbeispiel eines Keratometers.

Die Fig. 9 bis Fig. 12 zeigen verschiedene Implementierungen für Vorrichtungen zur Keratometrie.

Die Fig. 13A und die Fig. 13B zeigen eine alternative Implementierung für ein Keratometer mit Fixationsmarken.

Fig. 14 und Fig. 15 zeigen ein Mikroskop gemäß verschiedener nicht erfinderischer Ausführungsbeispiele.

Fig. 16 zeigt eine Kalibrierungsvorrichtung 150 für ein optisches Gerät 910 gemäß verschiedener nicht erfinderischer Ausführungsbeispiele.

Fig. 17 zeigt eine Flächenleuchte 170 umfassend eine Vorrichtung 100 gemäß verschiedener nicht erfinderischer Ausführungsbeispiele.

Fig. 18 zeigt ein Fenster gemäß einem nicht erfinderischen Ausführungsbeispiel.

Fig. 19 zeigt eine Beleuchtungsvorrichtung für ein aktives Augenimplantat gemäß einem nicht erfinderischen Ausführungsbeispiele.

Die Figuren zielen auf die Darstellung der zugrundeliegenden Prinzipien ab. Beispielsweise Oberflächenformen und Brechungen können daher schematisch angedeutet sein. Brechungen können beispielsweise übertrieben dargestellt oder vernachlässigt werden.

Zur Vermeidung von Wiederholungen werden gleiche oder einander entsprechende Elemente in verschiedenen Figuren mit dem gleichen Bezugszeichen bezeichnet und werden nicht mehrmals erläutert.

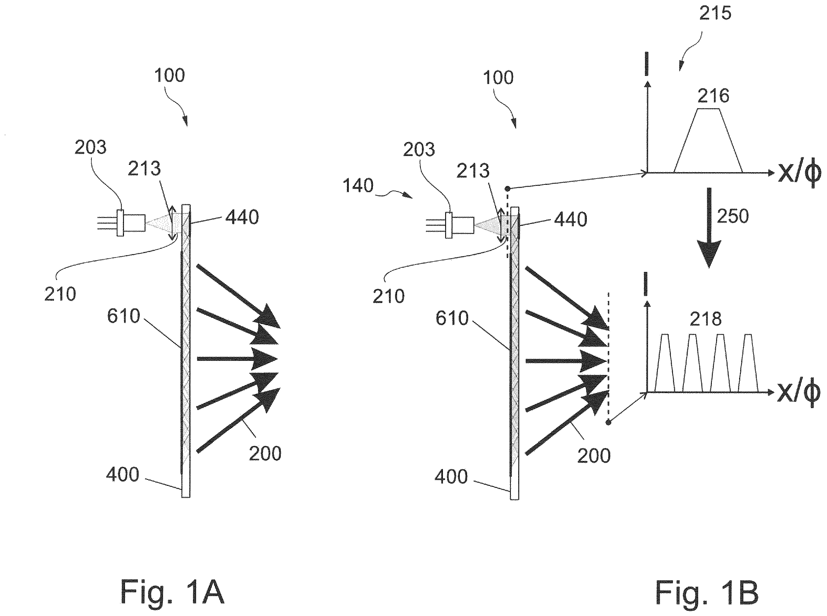

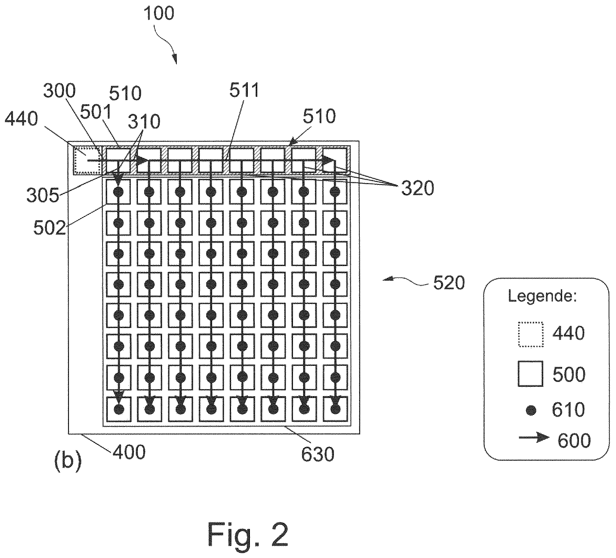

Zunächst werden zwei Ausführungsbeispiele der Vorrichtung anhand der Fig. 1A, Fig. 1B und Fig. 2 erläutert.

Fig. 1A zeigt eine Vorrichtung zur Erzeugung einer Leuchtverteilung gemäß einem Ausführungsbeispiel.

Die Vorrichtung 100 umfasst einen Lichtwellenleiter 400 mit einem Einkopplungselement 440. Die Vorrichtung 100 ist eingerichtet, Licht 210 von einer Lichtquelle 203 zu empfangen und Abgabelicht 610 als eine Leuchtverteilung 200 abzugeben. Die Leuchtverteilung kann verwendet werden, um ein Objekt zu beleuchten. Im gezeigten Beispiel der Fig. 1A und der Fig. 1B weist die Leuchtverteilung eine effektive Fokussierung auf, was durch die aufeinander zulaufenden Pfeile der Leuchtverteilung 200 angedeutet ist. In anderen Beispielen kann die Leuchtverteilung auch eine effektive Defokussierung aufweisen. In solchen Fällen würden die Pfeile, die die Leuchtverteilung 200 andeuten auseinanderlaufen und hätten einen virtuellen Ausgangspunkt auf der linken Seite des Lichtwellenleiters 400 der Fig. 1.

In anderen Beispielen kann die Leuchtverteilung 200 so eingerichtet sein, dass eine Vielzahl von Strahlen von unterschiedlichen Bereichen des Lichtwellenleiters 400 derart abgegeben werden, so dass das abgegebene Licht effektiv fokussiert und/oder effektiv defokussiert ist. Beispielsweise könnte Licht aus der oberen Hälfte des Lichtwellenleiters 400 eine effektive Fokussierung aufweisen und Licht aus der untern Hälfte des Lichtwellenleiters 400 eine effektive Defokussierung aufweisen.

Fig. 1B zeigt eine Vorrichtung entsprechend Fig. 1A gemäß einem anderen Ausführungsbeispiels.

In den Ausführungsbeispielen der Fig. 1A und der Fig. 1B wird das Licht 210 von einem Kollimator 213 kollimiert, bevor es auf das Einkopplungselement 440 trifft. Ein kollimierter Lichtstrahl kann für manche Einkopplungselemente vorteilhaft sein und beispielsweise die Einkopplungseffizienz steigern. Die Kollimierung kann, wie im Beispiel der Fig. 1B gezeigt, durch einen separaten Kollimator 213 erzielt werden. In anderen, nicht gezeigten, Ausführungsbeispielen kann die Kollimation auch durch das Einkopplungselement selbst erreicht werden.

Das Licht 210 weist ein Strahlprofil 215 mit einer ersten Modulation 216 auf. Die Vorrichtung 100 überführt das Licht 210 in die Leuchtverteilung 200. Die Leuchtverteilung 200 weist eine zweite Modulation 218 auf. Hierbei ist die Anzahl der Extremwerte der zweiten Modulation 218 größer als die Anzahl der Extremwerte der ersten Modulation 216 ist. Wie angedeutet kann die erste und/oder zweite Modulation in einem Ortsraum (Vektor "x") oder in einem Winkelraum ("φ") bestimmt werden. Mit anderen Worten kann die Modulation dadurch beobachtet werden, dass die Intensität als Funktion einer oder mehrerer Ortskoordinaten und/oder als Funktion einer oder mehrerer Winkelkoordinaten veränderlich ist, wobei die Anzahl der Extremwerte für die zweite Modulation 218 größer als für die erste Modulation 216 ist. Hierbei können die Koordinaten zweckdienlich normiert werden, beispielsweise bezogen auf einen Halbraum einer Einheitskugel wenn Licht von einer Seite auf ein Objekt einfällt oder auf einen Strahldurchmesser im Ortsraum.

Im Beispiel der Fig. 1B weist das Strahlprofil 215 eine Anzahl von einem Maximum und zwei Minima bezogen auf den Strahldurchmesser auf. Die zweite Modulation 218 der Leuchtverteilung 200 weist hingegen 4 Maxima bzw. 5 Minima auf. Somit weist die zweite Modulation 218 eine größere Anzahl von Extremwerten als die erste Modulation 216 auf.

Die Vorrichtung kann somit eine verhältnismäßig einfache Eingangslichtverteilung in eine komplexe Ausgangslichtverteilung umwandeln.

Fig. 2 zeigt eine Frontalansicht einer Vorrichtung entsprechend der Fig. 1A und/oder der Fig. 1B.

Im Ausführungsbeispiel der Fig. 2 sind die optischen Elemente im Lichtwellenleiter 400 als diskrete Einheiten ausgeführt. In anderen Ausführungsbeispielen können diese auch kontinuierlich ausgeführt sein, wie zuvor und nachfolgend beschrieben. Ein Einkopplungselement 440 ist mit einer Vielzahl von Replikationsbereichen 500 optisch gekoppelt, wobei die optische Kopplung 600 als Pfeil angedeutet ist. Jedes Element der Vielzahl von Replikationsbereichen 500 ist eingerichtet, ein zugehöriges Eingangslichtbündel mit einem Eingangsstrahlprofil zu empfangen und eine Vielzahl von zugehörigen Ausgangslichtbündeln mit jeweiligen Ausgangsstrahlprofilen bereitzustellen.

Es ist auch möglich, dass das Licht mehrfach mit einem Replikationsbereich interagiert, beispielsweise nach eine Totalreflektion im Lichtwellenleiter.

Insbesondere ist ein erster Replikationsbereich 501 der Vielzahl von Replikationsbereichen 500 mit dem mindestens einen Einkopplungselement 440 optisch gekoppelt 600, sodass der erste Replikationsbereich 501 eingerichtet ist, das Lichtbündel als das zugehörige Eingangslichtbündel 300 des ersten Replikationsbereichs zu empfangen. Der erste Replikationsbereich 501 ist ferner mit einem zweiten Replikationsbereich 502 der Vielzahl von Replikationsbereichen 500 optisch gekoppelt 600, sodass der zweite Replikationsbereich 502 eingerichtet ist, eines der Vielzahl von zugehörigen Ausgangslichtbündeln 310 des ersten Replikationsbereichs als das zugehörige Eingangslichtbündel des zweiten Replikationsbereichs 305 zu empfangen.

Die Vorrichtung 100 ist eingerichtet, Abgabelicht 610 von einer Menge der Vielzahl von Replikationsbereichen 500 aus dem Lichtwellenleiter 400 auszukoppeln, um die Leuchtverteilung 200, wie beispielsweise in den Fig. 1A und Fig. 1B gezeigt, bereitzustellen. Das Licht wird hierbei von einem Auskopplungsbereich 630 des Lichtwellenleiters 400 abgegeben.

Im gezeigten Ausführungsbeispiel der Fig. 2 umfasst die Vielzahl von Replikationsbereichen 500 eine erste Gruppe von Replikationsbereichen 510 (hervorgehoben durch Schraffierung), die jeweils miteinander optisch gekoppelt sind und das Licht jeweils an einzelne Gruppenelemente weiterleiten - mit Ausnahme des letzten Elements der ersten Gruppe - und in eine abweichende Richtung an andere Replikationsbereiche, die nicht zur ersten Gruppe von Replikationsbereichen gehören, weiter zu verteilen. Im gezeigten Ausführungsbeispiel gehören alle Replikationsbereiche entweder zu der ersten Gruppe von Replikationsbereichen oder zu der Menge der Vielzahl von Replikationsbereichen. In anderen Ausführungsbeispielen kann dies jedoch beliebig variiert werden.

Durch eine solche Vorrichtung 100 kann das am Einkopplungselement 440 bereitgestellte Licht vorteilhaft in eine Beleuchtungsverteilung überführt werden.

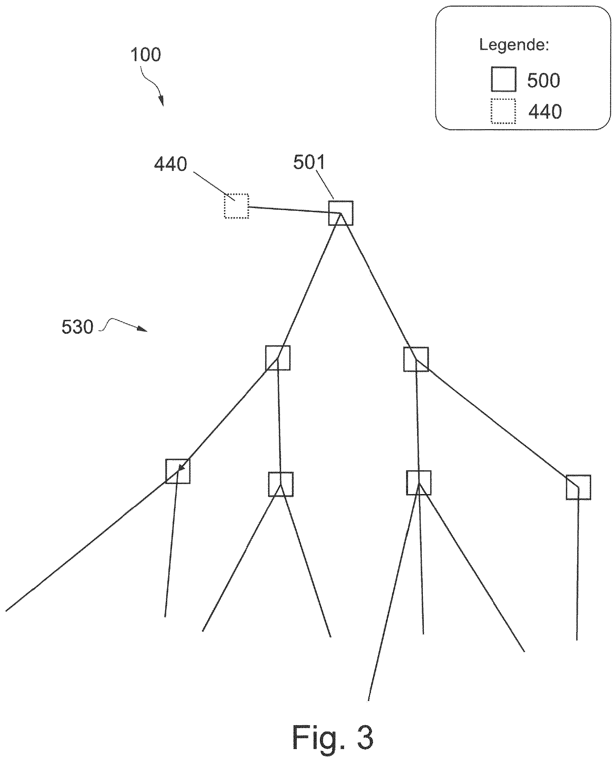

Fig. 3 zeigt ein Beispiel einer Vorrichtung mit einer Baumstruktur.

Fig. 3 zeigt hierbei eine Detailansicht einer Vorrichtung 100, die ebenfalls in einem Lichtwellenleiter angeordnet ist. Entsprechend der Darstellung von Fig. 2 wird eine Vielzahl von Replikationsbereichen 500 schematisch gezeigt, wobei ein erster Replikationsbereich 501 optisch mit einem Einkopplungselement 440 gekoppelt ist. Die weiteren Elemente der Vielzahl von Replikationsbereichen weisen eine Baumstruktur 530 auf.

Die Replikationsbereiche 500 können hierbei sowohl zur Weiterleitung von Licht im Lichtwellenleiter als auch zur Auskopplung von Licht aus dem Lichtwellenleiter, um eine Beleuchtungsverteilung zu erzeugen, eingerichtet sein. Durch die Baumstruktur wird der Freiheitsgrad von Leuchtverteilung, die von einer Vorrichtung 100 erzeugt werden kann, weiter gesteigert.

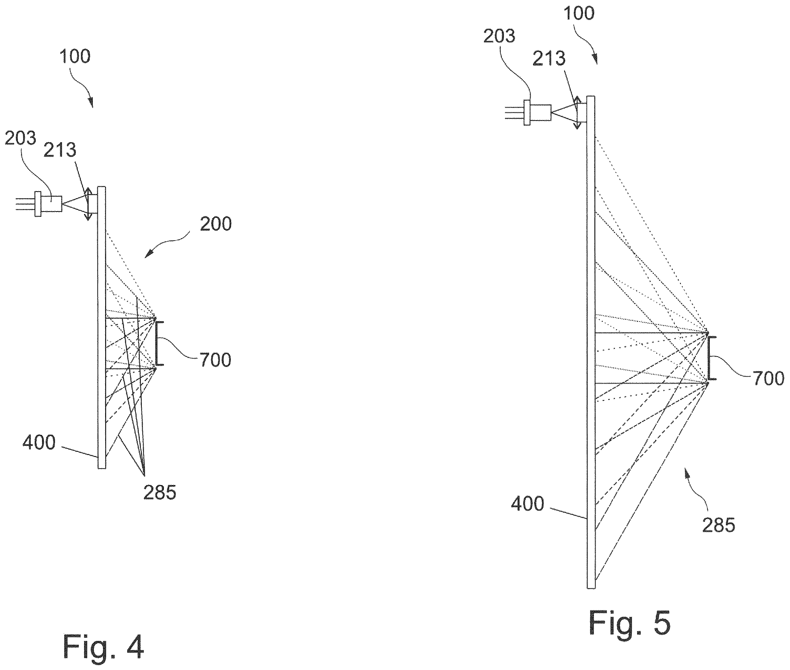

Fig. 4 zeigt ein weiteres Ausführungsbeispiel der Fig. 1A und der Fig. 1B.

Fig. 4 zeigt ein Ausführungsbeispiel einer Vorrichtung 100, wobei die Bezugszeichen mit der Vorrichtung der Fig. 1A und der Fig. 1B übereinstimmen. Die Vorrichtung 100 der Fig. 4 ist eingerichtet, eine Beleuchtungsverteilung 200 zur Beleuchtung eines Objekts 700 bereitzustellen. Im gezeigten Ausführungsbeispiel der Fig. 4 ist die Leuchtverteilung 200 so eingerichtet, dass eine Vielzahl von Strahlen 285 von unterschiedlichen Bereichen des Lichtwellenleiters 400 aus abgegeben werden.

Anders ausgedrückt umfasst die Leuchtverteilung unterschiedliche Lichtbündel, beispielsweise die Strahlen 285, die sich an dem Wellenleiter 400 überlappen. Ein Bereich des Wellenleiters 400 kann also Ausgangspunkt für Lichtbündel mit verschiedenen Richtungen sein. Dies kann beispielsweise durch mehrfachbelichtete Volumenhologramme, die als Replikationsbereiche und/oder Auskopplungselemente verwendet werden erreicht werden.

Hierdurch wird das empfangene Licht durch die Vorrichtung 100 effektiv auf das Objekt 700 fokussiert. Im gezeigten Ausführungsbeispiel sind die Strahlen 285 kollimiert und werden mit diskreten Winkelbereichen aus dem Lichtleiter 400 abgegeben.

Der Lichtwellenleiter 400 kann hierbei mindestens ein Auskopplungselement umfassen. Beispielsweise kann für jeder der Vielzahl von Strahlen 285 durch ein jeweiliges Auskopplungselement bereitgestellt werden. Hierbei kann das jeweilige Auskopplungselement jeweils Licht von mehreren Replikationsbereichen empfangen.

Fig. 5 zeigt eine weitere Vorrichtung 100. Diese entspricht im Wesentlichen der Vorrichtung der Fig. 4. Die Vorrichtung der Fig. 5 ist ebenfalls eingerichtet, das Objekt 700 unter gleichen Winkeln wie die Vorrichtung 100 der Fig. 4 zu beleuchten, jedoch bei einem größeren Abstand des Objekts 700 vom Lichtwellenleiter 400. Entsprechend ist die Vorrichtung 100 der Fig. 5 größer ausgeführt als die Vorrichtung 100 der Fig. 4.

Aus dem Vergleich der Fig. 4 mit der Fig. 5 ist zu ersehen, dass es erforderlich sein kann, den Durchmesser des Wellenleiters zu vergrößern, wenn ein Objekt mit einem gegebenen Durchmesser in einem größeren Abstand mit einer gleichen oder ähnlichen Leuchtverteilung beleuchtet werden soll.

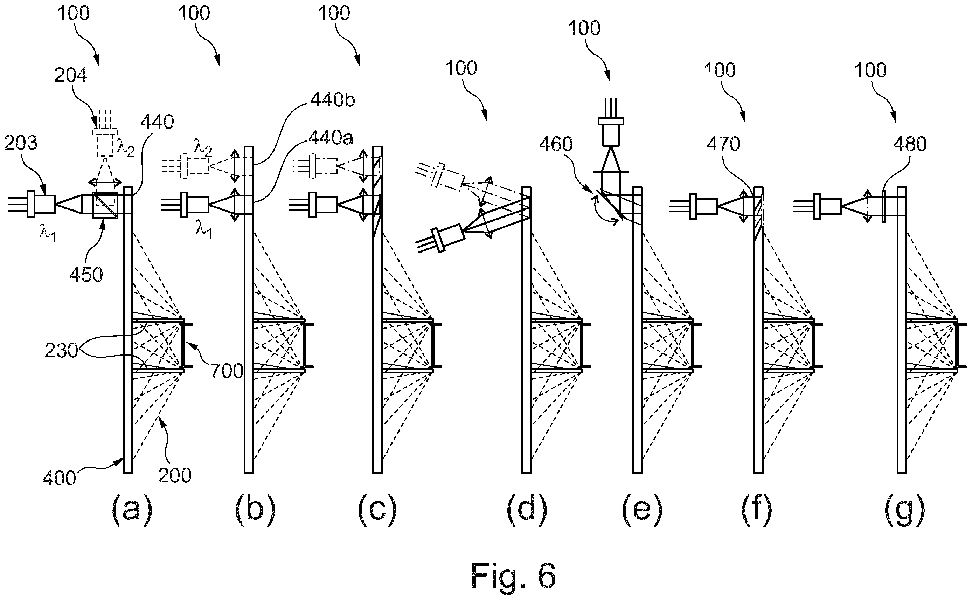

Fig. 6 zeigt Vorrichtungen gemäß verschiedener Ausführungsbeispiele, die mehrkanalig und/oder schaltbar sind.

Hierbei zeigen die Unterfiguren Fig. 6(a) bis Fig. 6(g) verschiedene Beispiele mehrkanaliger bzw. schaltbarer Vorrichtungen, die eingerichtet sind, unterschiedliche Leuchtverteilungen bereitzustellen.

Nachfolgend werden anhand der Vorrichtungen 100 bei (a) bis (g) verschiedene Konzepte mehrkanaliger Wellenleitersysteme beschrieben. Die Konzepte können sich hohe spektrale und/oder angulare Selektivität von diffraktiven Elementen, beispielsweise Volumenhologrammen oder anderen mikrostrukturierten optischen Elementen, zunutze machen, um mehrere Strahlenbündel unabhängig voneinander innerhalb desselben Volumens des Lichtleiters 400 übertragen zu können. Unter einer hohen spektralen Selektivität wird hierbei der Abfall der Effizienz des Elements beispielsweise um 50 % Halbwertsbreite, manchmal auch als Breite bei halber Höhe (Englisch: Full Width at Half Maximum, FWHM) bei Wellenlängenabweichungen von der Designwellenlänge beispielsweise <40 nm, beispielsweise <10 nm, verstanden.

Unter einer hohen angularen Selektivität wird ein Abfall der Effizienz des Elements um 50 % FWHM bei einer Abweichung des Strahleinfallswinkels von einem Designwinkel, für den das jeweilige optische Element ausgelegt ist, beispielsweise um ein zugehöriges Eingangslichtbündel aus diesem Winkel zu empfangen, von beispielsweise < 10°, beispielsweise < 2° verstanden. In solchen Fällen, aber nicht darauf beschränkt, können mehrere Strahlenbündel mit unterschiedlichen Richtungen und/oder Wellenlängen innerhalb des gleichen Volumens des Lichtwellenleiters 400 propagieren und können selektiv von zugeordneten, manchmal auch als "passenden" beschriebenen, optischen Elementen gekoppelt werden und weitergeleitet werden. Mit anderen Worten können innerhalb eines identischen Volumens des Lichtleiters 400 selektiv wirkende Replikationsbereiche bereitgestellt werden. Diese können in Superposition arbeiten und das Licht für unterschiedliche Charakteristiken, beispielsweise Einfallswinkel, in unterschiedliche Leuchtverteilungen umwandeln. Dies wird manchmal auch als Multiplexing beschrieben, beispielsweise als spektrales Multiplexing, wenn die optischen Elemente, beispielsweise Volumenhologramme, so eingerichtet sind, dass sie verschiedene Kopplungsverhalten für verschiedene spektrale Eigenschaften des Lichts aufweisen. Auch andere Arten von Multiplexing sind möglich, beispielsweise Winkel- oder polarisationsabhängiges Multiplexing, sowie Kombinationen davon.

Nachfolgend wird diese Grundidee am Beispiel von Seitenansichten von Vorrichtung 100 in Fig. 6 kurz erläutert. Es werden hierbei nur maximal zwei Lichtquellen beispielhaft gezeigt, dies ist natürlich nicht als einschränkend auszulegen, auch komplexere Systeme zum Beispiel mit mehr als zwei Lichtquellen sind möglich.

Die Vorrichtung bei (a) zeigt eine Vorrichtung 100, die eingerichtet ist, Licht von einer ersten Lichtquelle 203 mit einer ersten Wellenlänge λ1 und Licht von einer zweiten Wellenlänge λ2 von einer zweiten Lichtquelle 204 zu empfangen, und für jede empfangene Wellenlänge eine Leuchtverteilung 200 zu erzeugen. Im gezeigten Beispiel umfasst die Leuchtverteilung 200 eine Leuchtverteilung, welche sich aus der Leuchtverteilung 200 der Fig. 4 sowie einer Leuchtverteilung von Fixationsmarken 230 zusammensetzt. Ein solcher Aufbau kann den Vorteil haben, dass es möglich ist, mit demselben Lichtwellenleiter 400 in verschiedenen Wellenlängenbereichen für verschiedene Zwecke verschiedene Leuchtverteilungen bereitzustellen, im gezeigten Beispiel beispielsweise die Fixationsmarken 230 bei einer Wellenlänge λ2 der zweiten Lichtquelle 204 im sichtbaren Bereich und Infrarotlicht bei einer Wellenlänge λ1 der ersten Lichtquelle 203 im Infraroten.

Fig. 6(b) zeigt eine alternative Implementierung der Vorrichtung der Fig. 6(a) mit einem anders gestalteten Einkopplungselement 440. In diesem Ausführungsbeispiel umfasst das Einkopplungselement 440 zwei verschiedene Bereiche, wobei ein erster Einkopplungsbereich 440A eingerichtet ist, das Licht der ersten Lichtquelle 203 einzukoppeln, und ein zweiter Einkopplungsbereich 440B eingerichtet ist, das Licht der zweiten Lichtquelle 203 in den Lichtwellenleiter 400 einzukoppeln.

Die Fig. 6(c) bis Fig. 6(g) zeigen verschiedene Möglichkeiten zur Realisierung von schaltbaren Systemen und/oder Systemen, die mehrere Leuchtverteilungen in Überlagerung. Manchmal auch als Superposition bezeichnet, ermöglichen.

Im Beispiel der Fig. 6(c) sind die Lichtquellen 203, 204 lateral versetzt angeordnet und werden von Einkopplungselementen 440A, 440B an unterschiedlichen Stellen in den Lichtwellenleiter 400 eingekoppelt.

Die jeweiligen zugehörigen Einkopplungselemente 440A, 440B können hierbei so ausgestaltet sein, dass selbst bei gleichartigen Lichtquellen 203, 204 unterschiedliche Einkopplungen in den Lichtwellenleiter 400 erreicht werden, beispielsweise unterschiedliche Einkopplungswinkel. Somit kann die Vorrichtung 100 eingerichtet sein, zwei Beleuchtungsverteilungen, im gezeigten Beispiel eine jeweilige Beleuchtungsverteilung pro Lichtquelle, bereitzustellen. Diese Leuchtverteilungen können in manchen Beispielen unabhängig voneinander gewählt werden, beispielsweise aufgrund der zuvor beschriebenen Winkelselektivität und/oder Wellenlängenselektivität der verwendeten optischen Elemente.

Fig. 6(d) zeigt eine Variation der Fig. 6(c), wobei die beiden Lichtquellen 204, 203 unter unterschiedlichen Winkeln auf ein Einkopplungselement 440 treffen. Dieses ist eingerichtet, die beiden Lichtquellen unabhängig voneinander in den Lichtwellenleiter 400 einzukoppeln. Im Beispiel der Fig. 6 (e) ist nur eine Lichtquelle 203 vorhanden. Der Einfallwinkel des Lichtes der Lichtquelle 203 wird hierbei durch einen Scanspiegel 460 variiert, wodurch eine schaltbare Beleuchtungsverteilung entsteht. Im Beispiel der Fig. 6 (f) ist ein schaltbares optisches Element 470, beispielsweise ein schaltbares Hologramm, im Lichtwellenleiter 400 vorhanden. Auch hierdurch kann eine Superposition verschiedener Beleuchtungsverteilungen erreicht werden. Im Beispiel der Fig. 6 (g) verändert ein polarisationsveränderndes Element 480 die Polarisationseigenschaften des Lichts von der Lichtquelle 203. Die optischen Elemente der Vorrichtung 100 können polarisationsabhängige Eigenschaften aufweisen, sodass auch durch eine Variation der Polarisation des einfallenden Lichtes auf die Vorrichtung 100 unterschiedliche Beleuchtungsverteilungen herbeigeführt werden können.

Die in den Fig. 6 (a) bis Fig. 6 (g) gezeigten Beispiele können auch miteinander kombiniert werden. So können beispielswiese verschiedene Lichtquellen mit unterschiedlichen Polarisationsrichtungen - entsprechend dem Beispiel der Fig. 6(g) - mit einem Scanspiegel - wie in Fig. 6(e) gezeigt - kombiniert werden. Aber auch beliebige andere Kombinationen der gezeigten Elemente und Vorgehensweisen sind möglich.

Im Zusammenhang mit den nachfolgenden Figuren werden verschiedene Anwendungsmöglichkeiten der bislang gezeigten Vorrichtungen weiter illustriert.

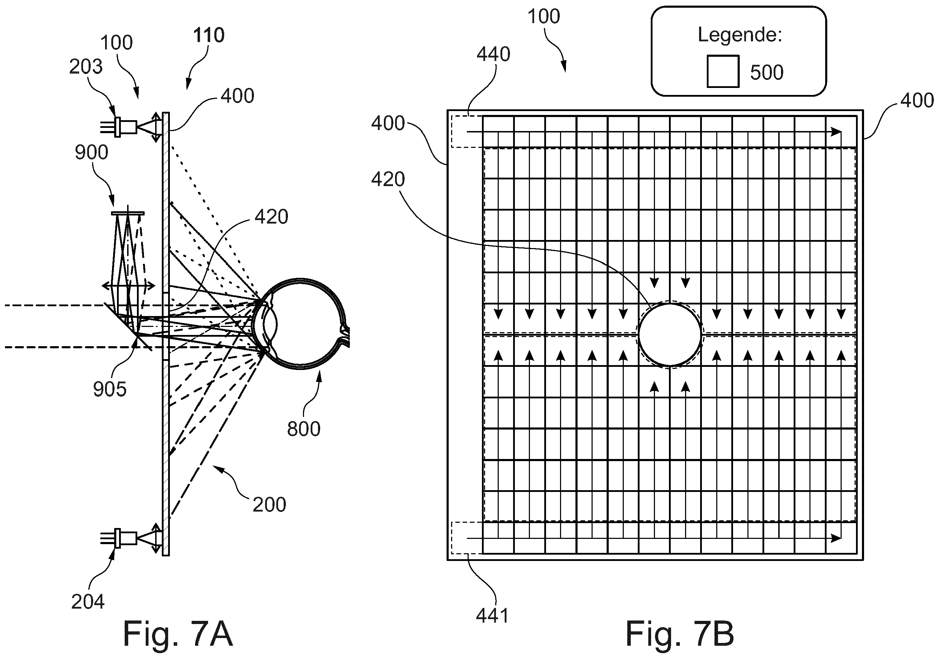

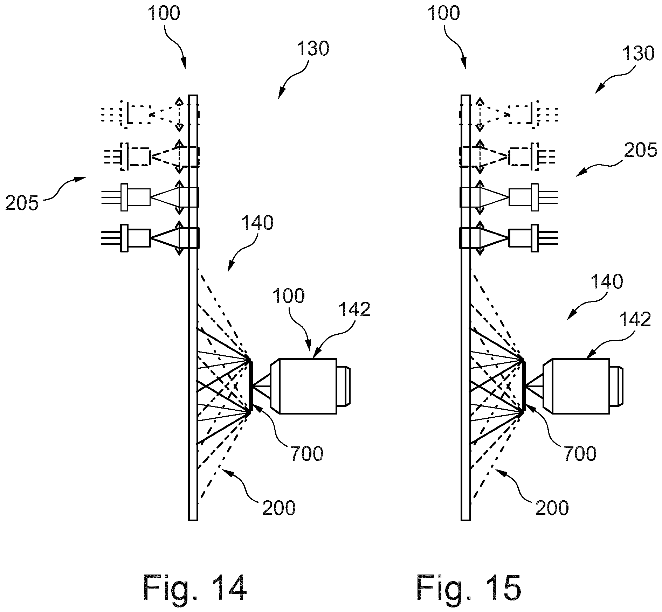

Die Fig. 7A bis Fig. 13B zeigen Vorrichtungen, die zur Bereitstellung eines Keratometers verwendet werden. Die Fig. 14 und die Fig. 15 zeigen verschiedene Aufbauten für ein Mikroskop.

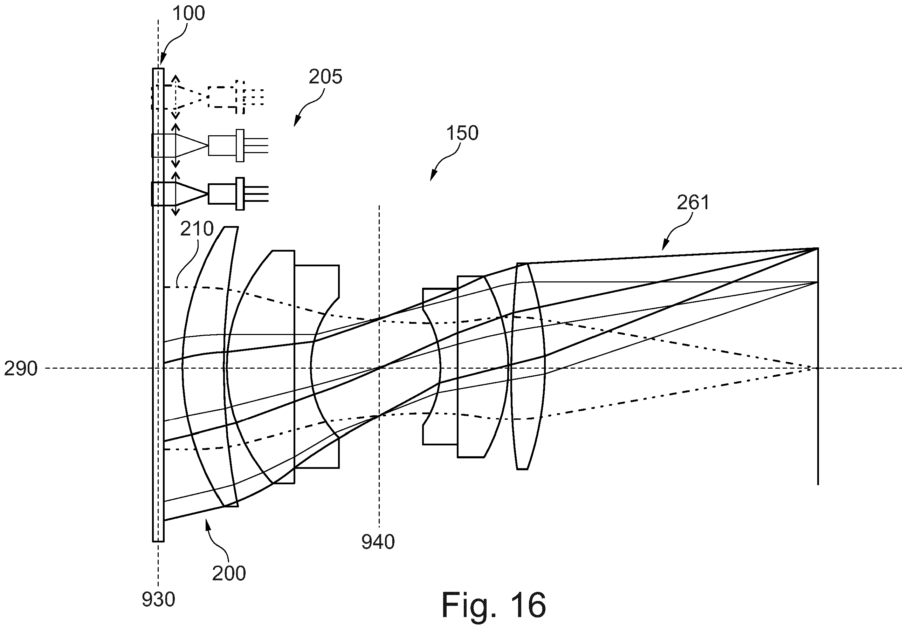

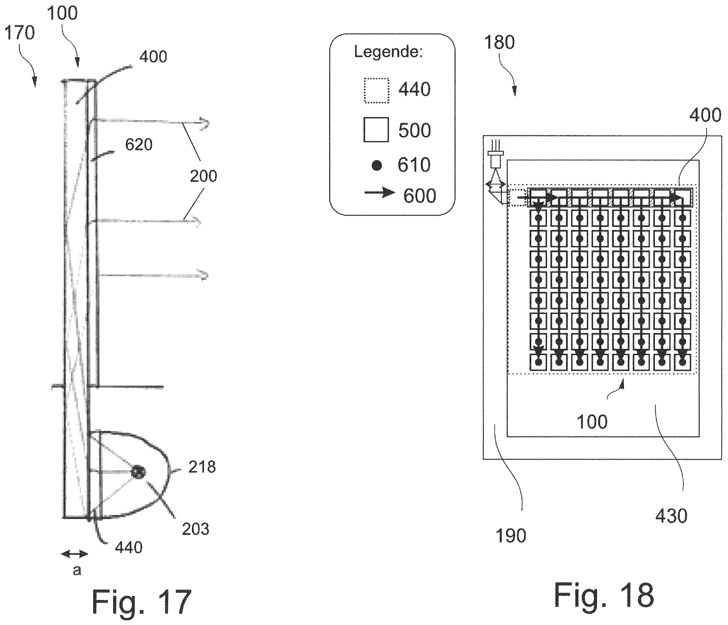

Die Fig. 16 zeigt eine Kalibrierungsvorrichtung für ein optisches Gerät, die Fig. 17 und die Fig. 18 zeigen Vorrichtungen zur Flächenbeleuchtung sowie ein Fenster für ein Gebäude.

Fig. 7A zeigt eine Seitenansicht eines erfindungsgemäßen Keratometers. Fig. 7B zeigt eine Frontalansicht.

Mit einer erfindungsgemäßen Vorrichtung 100 wird eine Leuchtverteilung 200 zur keratometrischen Vermessung der Hornhaut eines Auges 800 bereitgestellt. Das von der Hornhaut des Auges 800 reflektierte Licht wird von einer Detektionsvorrichtung 900 entlang eines Nachweisstrahlengangs 905 nachgewiesen und kann anschließend analysiert werden, um auf die Topologie der Hornhaut zu schließen. Der Lichtwellenleiter 400 der Vorrichtung 100 weist eine Aussparung 420 auf. Um trotz der Aussparung 420 eine zur Keratometrie geeignete Beleuchtungsverteilung 200 zu erreichen, die möglichst das gesamte zu untersuchende Auge beleuchtet, wird das Licht von zwei Lichtquellen 203, 204 bereitgestellt und von zwei Einkopplungselementen 440, 441 eingekoppelt. Ausgehend von den jeweiligen Einkopplungselementen 440, 441, wird das Licht über eine Vielzahl von Replikationsbereichen repliziert und in Richtung des Auges 800 als Beleuchtungsverteilung 200 ausgekoppelt.

In dem geigten Beispiel des Kartometers ist die Flächennormale des Lichtwellenleiters parallel zu einer Hauptsehachse des Auges 800 angeordnet. In anderen Ausführungsbeispielen kann die Normale des Lichtwellenleiters aber auch gerade nicht parallel zu der Hauptsehachse des Auges 800 angeordnet sein. Hierdurch können beispielsweise Reflexe reduziert oder vermieden werden.

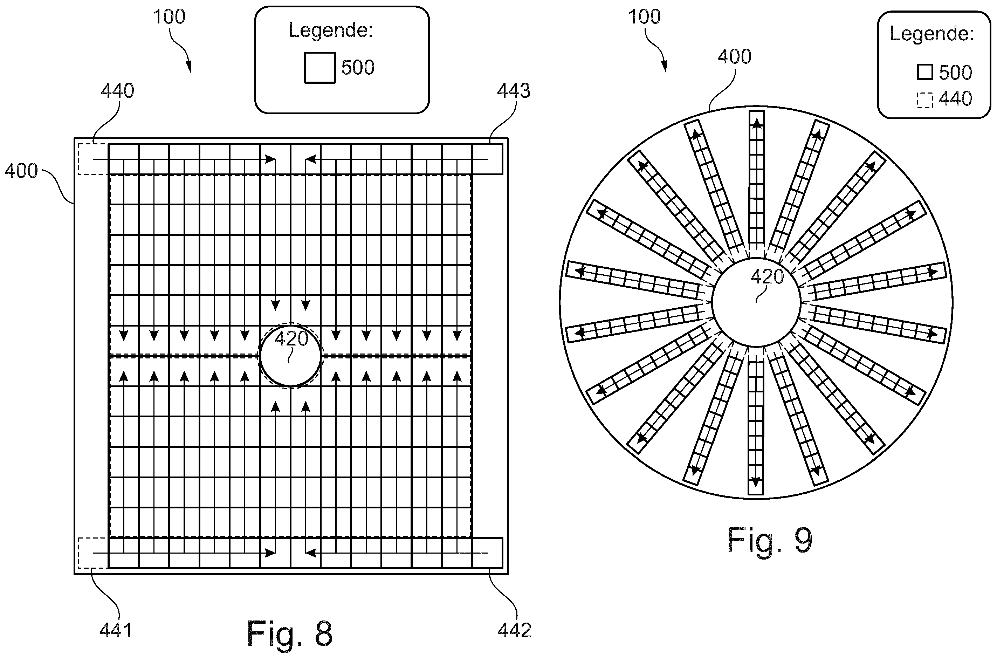

Fig. 8 zeigt ein weiteres Beispiel eines Keratometers.

In der Vorrichtung 100 der Fig. 8 sind vier Einkopplungselemente 440 bis 443 vorhanden. Die Vielzahl von Replikationsbereichen 500 sind hierbei so miteinander gekoppelt, dass eine Beleuchtungsverteilung wie die Beleuchtungsverteilung 200 der Fig. 7(a) durch die Vielzahl von Replikationsbereichen 500 bereitgestellt werden kann.

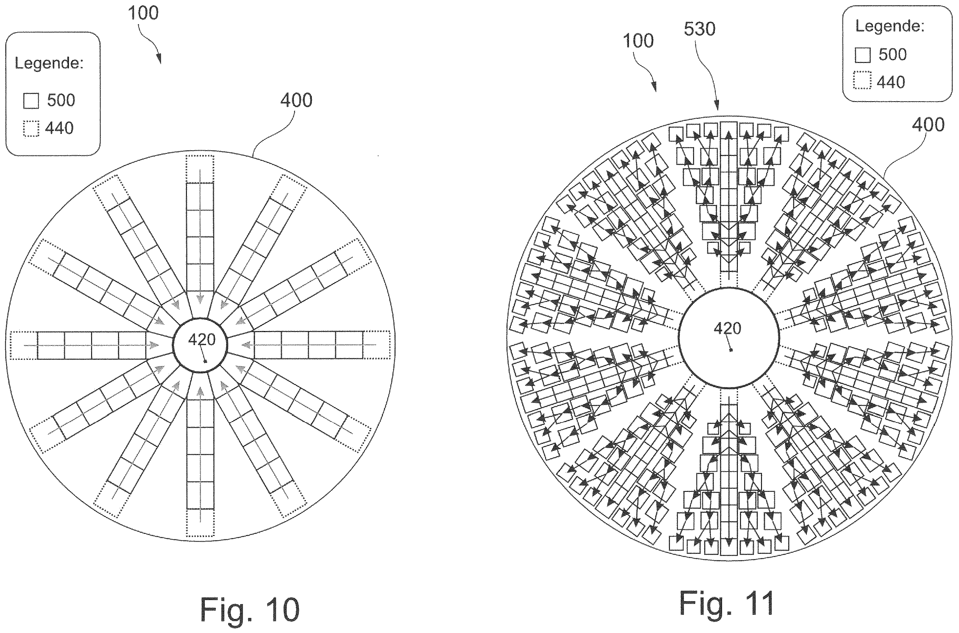

Die Fig. 9 bis Fig. 12 zeigen verschiedene Implementierungen für Vorrichtungen zur Keratometrie. Die Vorrichtungen der Fig. 9 bis Fig. 11 weisen jeweils eine Aussparung 420 im Zentrum eines runden Lichtwellenleiters 400 auf. Im Beispiel der Fig. 9 wird das Licht nahe der Aussparung 420 von einer Vielzahl von Einkopplungselementen 440 empfangen und in Reihe an eine Vielzahl von Replikationsbereichen weitergeleitet, die ebenfalls zur Auskopplung des Lichts in Richtung des Auges 800 dienen. Fig. 10 zeigt eine ähnliche Anordnung, wobei die Vielzahl von Einkopplungselementen 440 nicht in räumlicher Nähe zur Aussparung 420, sondern in der Nähe des Randes des Lichtwellenleiters 400 angeordnet ist. Das von der Vielzahl der Einkopplungselemente 440 empfangene Licht wird in Reihenschaltung an die Vielzahl von Replikationsbereichen weitergeleitet. Die Replikationsbereiche können hierbei beliebig geformt sein. Im gezeigten Beispiel der Fig. 10 sind diese rechteckig geformt, in der Nähe der Aussparung 420 haben diese jedoch eine komplexere Form, wobei auch andere Formen möglich sind und die gezeigten Formen nur beispielhaft sind.

Im Ausführungsbeispiel der Fig. 11 sind die Einkopplungselemente 440 abermals entsprechend der Fig. 9 in der Nähe der Aussparung 420 angeordnet. Die optische Kopplung der Vielzahl der Replikationsbereiche weist nun eine Baumstruktur 530 auf. Hierdurch kann eine homogene Ausleuchtung des Auges erreicht werden.

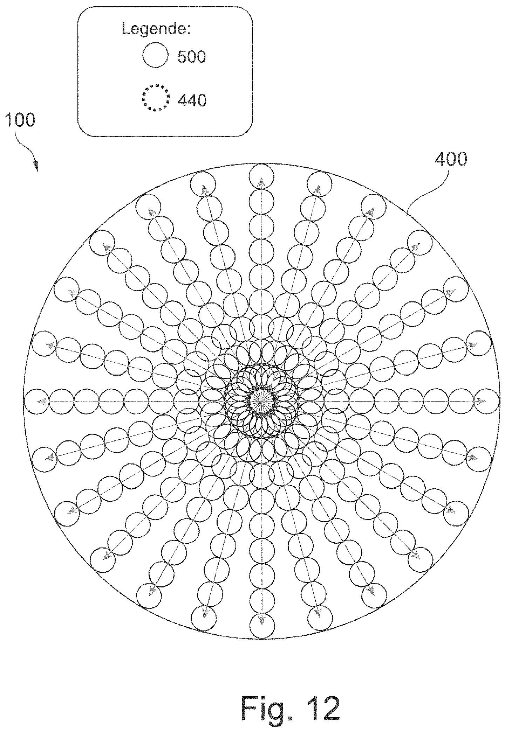

Im Ausführungsbeispiel der Fig. 12 wird das Licht über ein einzelnes Einkopplungselement 440 im Zentrum des Lichtwellenleiters 400 eingekoppelt. Die Vielzahl von Replikationsbereichen 500 ist im Ausführungsbeispiel der Fig. 12 kreisförmig geformt und überlappt einander in der Frontalansicht. Dies kann durch einen Versatz innerhalb des Lichtwellenleiters oder durch eine volumetrische Überlappung, beispielsweise bei Volumenhologrammen, wie bereits zuvor beschrieben, erreicht werden. Aufgrund der Winkelselektivität von manchen diffraktiven Elementen kann es so möglich sein, trotz des Überlapps der Vielzahl von Auskopplungsbereichen 500 eine wohldefinierte Lichtverteilung 200 bereitzustellen.

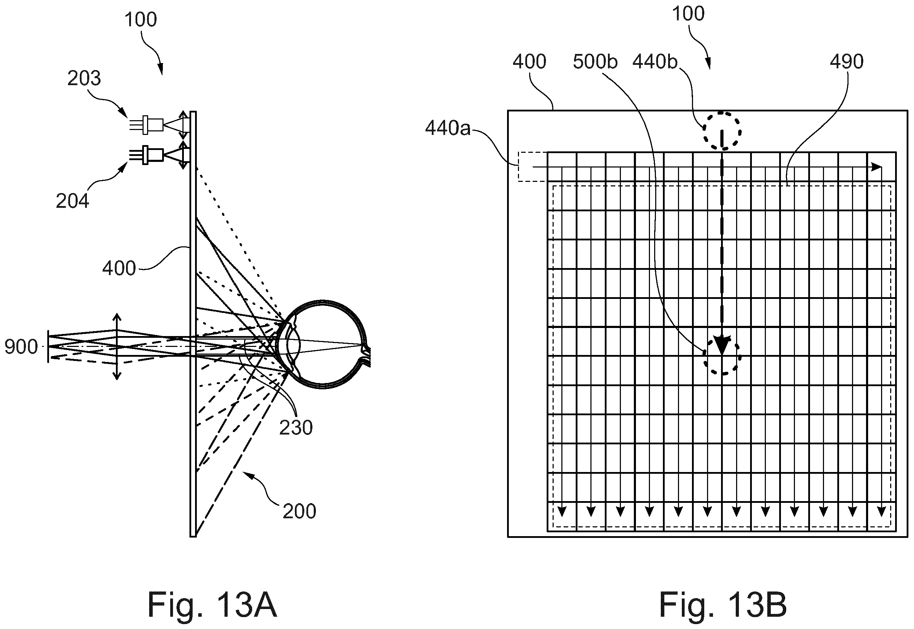

Die Fig. 13A und Fig. 13B zeigen eine alternative Implementierung für ein Keratometer mit Fixationsmarken.

Im Ausführungsbeispiel der Fig. 13A und Fig. 13B weist der Lichtwellenleiter 400 keine Aussparung auf. Die Beobachtung durch die Detektionsvorrichtung 900 erfolgt durch den Lichtwellenleiter 400 hindurch. Das Keratometer 120 kann eine Vorrichtung 100 gemäß einem Ausführungsbeispiel der Fig. 6 umfassen. Gezeigt ist hier ein Ausführungsbeispiel gemäß der Fig. 6(b). Wie im Zusammenhang mit der Fig. 6 (b) beschrieben, erzeugen die zwei Lichtquellen 203, 204 zwei Beleuchtungsverteilungen 200, wobei die erste Lichtquelle 203 die für die Keratometrie erforderliche Beleuchtungsverteilung 200 im Infraroten bereitstellt, und eine im Sichtbaren emittierende Lichtquelle 204 Fixationsmarken 230 bereitstellt. In der Seitenansicht der Fig. 13B ist zu erkennen, dass die Vorrichtung 100 hierfür ein Einkopplungselement 440a für das Infrarotlicht aufweist, welches entsprechend dem Ausführungsbeispiel der Fig. 2 wirkt. Für die Fixationsmarken wird das Licht der Lichtquelle 204 von einem Einkopplungselement 440b eingekoppelt und von einem Replikationsbereich 500b ausgekoppelt, um die Fixationsmarken 230 als Beleuchtungsverteilung bereitzustellen.

Nachfolgend wird ein weiteres Anwendungsbeispiel aus dem Bereich der Mikroskopie erläutert.

Die Fig. 14 und die Fig. 15 zeigen ein Mikroskop gemäß verschiedener Ausführungsbeispiele.

Das Mikroskop 130 weist eine Probenbeleuchtungsvorrichtung 140 und ein Okular 142 auf. Diese umfasst eine Vorrichtung 100 gemäß den vorherigen Ausführungsbeispielen und ist eingerichtet, eine Beleuchtungsverteilung auf einer Probe 700 zu erzeugen. Die Beleuchtungsverteilung kann insbesondere ein Muster auf der Probe 700 sein. Hierfür kann die Vorrichtung 100 eingerichtet sein, Licht von einer vielfach modulierbaren Lichtquelle 205 zu empfangen. Das empfangene Licht kann dann in eine Leuchtverteilung 200 umgewandelt werden. Hierbei kann die vielfach modulierbare Lichtquelle sowohl auf der mikroskopabgewandten Seite, wie in Fig. 14 gezeigt, angeordnet sein, sie kann aber auch auf der mikroskopzugewandten Seite, wie in Fig. 15 gezeigt, angeordnet sein. Durch die Gestaltungsfreiheit der Vorrichtung 100 kann insbesondere eine winkelvariable Beleuchtung der mikroskopischen Probe 700 bereitgestellt werden. Hierdurch können Beleuchtungsanforderungen in der Mikroskopie, wie sie beispielsweise in der Fourierptychographie auftreten, mit verringertem Aufwand und/oder gesteigerter Qualität erfüllt werden. Die modulierbaren Lichtquellen können hierbei zeitselektiv geschaltet werden.

Häufig müssen nicht Strahlenbündel einzeln geschaltet werden, sondern es können zur Beschleunigung der Bildaufnahme Gruppen von Strahlenbündeln an und ausgeschaltet werden. Jede dieser Gruppen von gemeinsam geschalteten Strahlenbündeln kann auch als eine Leuchtverteilung angesehen werden. Hierbei kann eine Leuchtverteilung von einer Lichtquellenanordnung bereitgestellt werden, wie zuvor und nachfolgend beschrieben. Manche Bildoptimierungsverfahren, können beispielsweise bereits mit Licht von 4 separat schaltbaren Beleuchtungsverteilungen realisiert werden. Hierfür ist es jedoch erforderlich, dass jede der vier schaltbaren Leuchtverteilungen Licht aus mehreren diskreten Richtungen auf die Probe sendet. Solche schaltbaren Leuchtverteilungen, auch für weniger oder mehr als 4 Schaltzustände, können erfindungsgemäß bereitgestellt werden.

Ein weiteres Anwendungsbeispiel wird nachfolgend erläutert.

Fig. 16 zeigt eine Kalibrierungsvorrichtung 150 für ein optisches Gerät 910 gemäß verschiedener Ausführungsbeispiele.

Die Vorrichtung gemäß verschiedener Ausführungsbeispiele kann vorteilhaft zur Kalibrierung und Justage von abbildenden optischen Systemen, beispielsweise von Objektiven, Anwendung finden. Dies kann insbesondere im Zusammenhang mit schwer zugänglichen optischen Geräten vorteilhaft sein, beispielsweise Objektive oder andere Bildgebungssysteme, welche sich im Inneren von Maschinen befinden oder die unter schwierigen Umgebungsbedingungen, beispielsweise unter Wasser oder im Weltraum, verwendet werden.

Im Ausführungsbeispiel der Fig. 16 ist ein planares Lichtwellenleitersystem 400 direkt im Strahlengang 290 eines optischen Geräts 910 angeordnet. Die Vorrichtung 100 ist eingerichtet, Licht von einer vielfach modulierbaren Lichtquelle 205 zu empfangen und eine Beleuchtungsverteilung bereitzustellen. Die vielfach modulierbare Lichtquelle 205 kann jedoch auch eine nicht-modulierbare Lichtquelle sein, beispielsweise wenn nur eine einzige Beleuchtungsverteilung benötigt wird, beispielsweise ein einzelnes Testbild.

Diese Leuchtverteilung 200 kann nun verwendet werden, um die Kalibrierung des optischen Geräts 910 durchzuführen. Hierfür können insbesondere verschiedene Wellenlängen von Licht von der vielfach modulierbaren Lichtquelle 205 gleichzeitig oder zeitlich sequenziell bereitgestellt werden. Zusätzlich oder alternativ kann die Leuchtverteilung 200 so bereitgestellt werden, dass das Licht 210 den Lichtwellenleiter so verlässt, dass es unter wohldefinierten Einfallslichtwinkeln in das optische Gerät 910 einfällt. Hierdurch kann eine Kalibrierung des optischen Geräts 910 vorteilhaft durchgeführt werden.

Durch die hohe Winkelselektivität der optischen Elemente im Lichtwellenleiter 400 wird gleichzeitig der normale Betrieb des optischen Geräts 910 nicht oder nur vernachlässigbar beeinflusst. Im gezeigten Ausführungsbeispiel der Fig. 16 befindet sich die Kalibrierungsvorrichtung 150 in einer ersten Installationsposition 930. Eine solche Installationsposition außerhalb der optischen Elemente des optischen Geräts 910 kann insbesondere eine einfache austauschbare Installation, beispielsweise als ein Filterelement, ermöglichen.

Alternativ oder zusätzlich kann die Kalibrierungsvorrichtung 150 an einer Position innerhalb von optischen Elementen des optischen Geräts 940 installiert werden. Hierdurch kann eine Teilkalibrierung einzelner optischer Elemente effizient ermöglicht werden.

Indem die Leuchtverteilung nicht vor, sondern z.B. zwischen Baugruppen des Objektivs bereitgestellt wird, lassen sich neue Konzepte zur Prüfung und zur Justage von optischen Geräten realisieren. In solchen Fällen kann die Leuchtverteilung beispielsweise eine Nominalwellenfront des optischen Geräts darstellen, die durch die vorgelagerten Objektivsubgruppen und ein Standardtestobjekt entstünde.

Die Installation solcher Kalibrierungsvorrichtungen wie der gezeigten Kalibrierungsvorrichtung 150 kann fest oder temporär sein. Beispielsweise kann die Kalibrierungsvorrichtung für eine Kalibrierung in den Strahlengang eingefahren werden. In anderen Ausführungsformen kann sie auch fest im Strahlengang verbleiben. Hierbei kann es vorteilhaft sein, dass aufgrund der starken Wellenlängen- und/oder Winkelselektivität der verwendeten Vorrichtungen der Einfluss auf den Strahlengang des optischen Geräts durch die Kalibrierungsvorrichtung gering sein kann. In solchen Fällen, in denen die Vorrichtung fest im Strahlengang installiert ist, kann die Vorrichtung beim optischen Design des optischen Geräts berücksichtigt werden. Durch die hohe angulare und spektrale Selektivität der Vorrichtung im Lichtwellenleiter können für einen ausgewählten Feldpunkt des optischen Geräts nur schmale spektrale Teilbänder durch einen Lichtwellenleiter herausgefiltert werden, so dass die Funktionsweise des optischen Geräts nicht oder nur minimal beeinflusst wird. Bei diesen Wellenlängenbändern können die Justagemarken und Testmuster mit hoher Effizienz eingespiegelt werden.

Die von der Vorrichtung angebotenen Testmuster können in verschiedenen Entfernungen, Wellenlängen, Positionen und Formen dargestellt werden. Dabei ist es möglich, mit einer Strahlungsquelle mehrere Testmuster gleichzeitig zu erzeugen. Es können aber auch mehrere Lichtquellenanordnungen verwendet werden und/oder andere der beschriebenen Vorgehensweisen angewendet werden, um zusätzlich oder alternativ schaltbare Muster zu erzeugen.

Fig. 17 zeigt eine Flächenleuchte 170 umfassend eine Vorrichtung 100 gemäß verschiedener Ausführungsbeispiele. Im Ausführungsbeispiel der Fig. 17 wird Licht von einer ersten Lichtquelle 203 von einem Parabolreflektor 218 an ein Einkopplungselement 450 der Vorrichtung 100 bereitgestellt. Wie zuvor beschrieben, wird das eingekoppelte Licht mittels einer Vielzahl von Replikationsbereichen innerhalb des Lichtwellenleiters 400 weitergeleitet. Im Ausführungsbeispiel der Fig. 17 wird die Leuchtverteilung 200 von einem Auskoppelement 620 bereitgestellt. Aber auch andere Möglichkeiten, die Lichtverteilung 200 bereitzustellen, wie zuvor beschrieben, können verwendet werden. Hierbei können insbesondere sehr geringe Abmessungen A des Lichtwellenleiters 400 realisiert werden, wobei gleichzeitig ein hoher Freiheitsgrad von der Leuchtverteilung 200 zur Verfügung steht. Im gezeigten Ausführungsbeispiel der Fig. 17 wird kollimiertes Licht als Leuchtverteilung 200 bereitgestellt, aber auch andere, mehr komplexe Leuchtverteilungen können erzeugt werden.

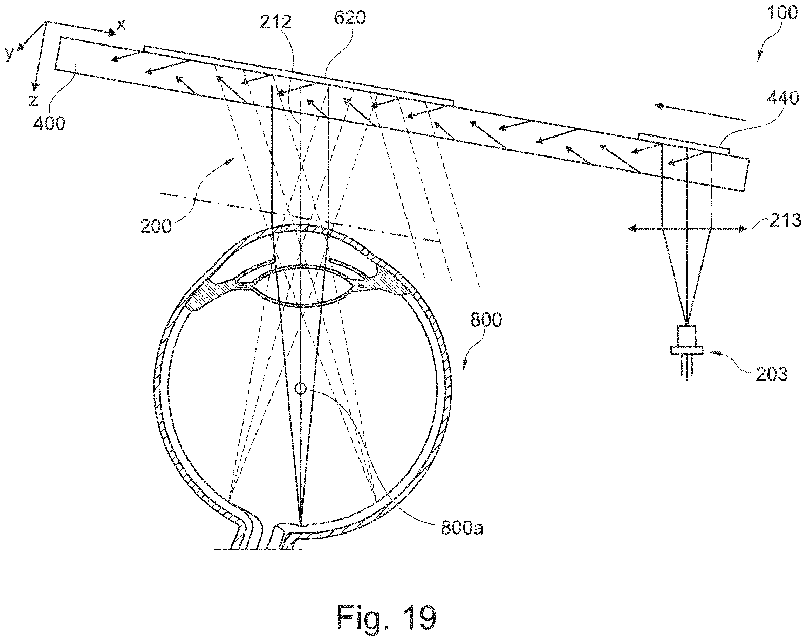

Fig. 18 zeigt ein Fenster 180. Das Fenster 180 umfasst ein Fensterglas 430, mit einem Lichtwellenleiter 400 gemäß verschiedener Ausführungsbeispiele der zuvor beschriebenen Vorrichtung. Das Fenster 180 umfasst ferner einen Fensterrahmen 190. Innerhalb des Fensterrahmens 190 und im Ausführungsbeispiel der Fig. 18 unsichtbar, ist eine Lichtquelle 203 angeordnet. Die Lichtquelle 203 ist eingerichtet, Infrarotlicht bereitzustellen und dieses an das mindestens eine Einkopplungselement 450 der Vorrichtung 100 bereitzustellen. Die Vorrichtung 100 im Lichtwellenleiter erzeugt eine Beleuchtungsverteilung 200. Hierbei kann die Leuchtverteilung 200 als eine Wärmequelle, beispielsweise als eine Heizung für ein Zimmer in einem Haus, auf mindestens einer Seite des Fensters bereitgestellt werden. Das von der Lichtquelle 203 bereitgestellte Infrarotlicht kann insbesondere in einem Spektralbereich von 1 bis 10 $\mu$m einem Intensitätsmaximum aufweisen. Fig. 19 zeigt eine Beleuchtungsvorrichtung für ein aktives Augenimplantat gemäß einem Ausführungsbeispiel.

Die Vorrichtung 100 ist eingerichtet, Licht von einer Lichtquelle 203 an ein aktives Augenimplantat im Auge 800 eines Benutzers bereitzustellen. Bei diesem Anwendungsbeispiel wird das Licht von einem Einkopplungselement 440 in den Lichtwellenleiter 400 eingekoppelt. Der Lichtwellenleiter 400 ist hierbei schräg gegenüber dem Auge angeordnet. Dies kann ästhetische Vorteile bieten, wenn der Lichtwellenleiter in einer Brille angeordnet ist.

Das Licht propagiert im Lichtwellenleiter 440 in Totalreflexion und wird von einem Auskopplungselement 620 ausgekoppelt und stellt die Leuchtverteilung 200 an das Auge und somit an das Augenimplantat bereit. Im gezeigten Beispiel wird das Licht als eine Vielzahl von kollimierten Strahlen, beispielsweise kollimiertes Strahlbündel 212, bereitgestellt. Die Vielzahl von kollimierten Strahlen sind hierbei effektiv fokussiert, da sie vom Lichtwellenleiter 400 ausgehend eine größere Ausgangsfläche an dem Lichtwellenleiter durchlaufen als in einer gedachten Fokussierebene (angedeutet als punkt-gestrichelte Linie vor dem Auge 800). Durch die Vielzahl von ausgekoppelten Strahlen kann auch bei einer Drehung des Auges um den Augendrehpunkt 800a sichergestellt werden, dass unabhängig von der Blickrichtung das Augenimplantat mit Licht versorgt ist.

**Patentansprüche**

1. Vorrichtung (100) zur Erzeugung einer Leuchtverteilung (200) zum Beleuchten eines Objekts (700), umfassend: einen Lichtwellenleiter (400), umfassend folgende optische Elemente: mindestens ein Einkopplungselement (440; 441-443), eingerichtet zum Einkoppeln von Licht (210) in den Lichtwellenleiter (400) als ein Lichtbündel mit einem zugehörigen Strahlprofil (215),

   eine Vielzahl von Replikationsbereichen (500) zur Replikation des Lichtbündels, jeweils eingerichtet, mindestens ein zugehöriges Eingangslichtbündel mit einem Eingangsstrahlprofil zu empfangen und eine Vielzahl von zugehörigen Ausgangslichtbündeln mit jeweiligen Ausgangsstrahlprofilen bereitzustellen, wobei die Vielzahl von Replikationsbereichen (500) Volumenhologramme sind, wobei die Volumenhologramme gerade oder schräg innerhalb des Lichtwellenleiters (400) angeordnet sind und/oder mehrfachbelichtet sind,

   wobei mindestens ein erster Replikationsbereich (501) der Vielzahl von Replikationsbereichen (500) mit einem zweiten Replikationsbereich (502) der Vielzahl von Replikationsbereichen (500) optisch gekoppelt (600) ist, sodass der zweite Replikationsbereich eingerichtet ist, mindestens eines der Vielzahl von zugehörigen Ausgangslichtbündeln (310) des ersten Replikationsbereichs als das zugehörige Eingangslichtbündel des zweiten Replikationsbereichs (305) zu empfangen und wobei der erste Replikationsbereich mit dem mindestens einen Einkopplungselement (440; 441-443) optisch gekoppelt (600) ist, um das Lichtbündel als das zugehörige Eingangslichtbündel (300) des ersten Replikationsbereichs (501) zu empfangen, wobei die Vorrichtung (100) eingerichtet ist, Abgabelicht (610) von einer Menge der Vielzahl von Replikationsbereichen aus dem Lichtwellenleiter (400) auszukoppeln, um die Leuchtverteilung (200) bereitzustellen,

   wobei die Vielzahl von Replikationsbereichen (500) eine erste Gruppe von Replikationsbereichen (510) umfasst, die jeweils miteinander optisch gekoppelt sind und wobei die Replikationsbereiche der ersten Gruppe von Replikationsbereichen (510) jeweils eingerichtet sind:

   mindestens ein erstes zugehöriges Ausgangsbündel der Vielzahl von Ausgangslichtbündeln an einen anderen Replikationsbereich der ersten Gruppe von Replikationsbereichen (510) bereitzustellen, und mindestens ein zweites zugehöriges Ausgangsbündel der Vielzahl von Ausgangslichtbündeln nicht an einen anderen Replikationsbereich der ersten Gruppe von Replikationsbereichen (510) bereitzustellen, um eine Menge von Abgabebündeln (320) der ersten Gruppe von Replikationsbereichen (510) zu erhalten, und

   wobei die Vielzahl von Replikationsbereichen (500) eine zweite Gruppe von Replikationsbereichen (520) umfasst, die jeweils miteinander optisch gekoppelt sind und von denen eine Teilmenge eingerichtet ist, die Menge von Abgabebündeln (320) der ersten Gruppe von Replikationsbereichen (510) als jeweilige Eingangslichtbündel zu empfangen.

2. Vorrichtung (100) nach Anspruch 1, wobei der Lichtwellenleiter (400) eingerichtet ist, das Licht mit einer ersten Modulation (215) zu empfangen, wobei die Vorrichtung (100) so eingerichtet ist, dass die Leuchtverteilung (200) eine zweite Modulation (218) aufweist, wobei die zweite Modulation (218) eine größere Anzahl an Extremwerten als die erste Modulation (216) aufweist.

3. Vorrichtung (100) nach Anspruch 1 oder Anspruch 2, wobei die Vorrichtung (100) keinen räumlichen Lichtmodulator, der eingerichtet ist, in den Lichtwellenleiter einzukoppelndes Licht basierend auf Daten zu modulieren, umfasst.

4. Vorrichtung (100) nach einem der vorherigen Ansprüche, wobei zumindest eine Teilmenge der Vielzahl von Replikationsbereichen eine Teilleuchtverteilung (200) der Leuchtverteilung (200) bereitstellt, die eine effektive Fokussierung aufweist.

5. Vorrichtung (100) nach einem der vorherigen Ansprüche, wobei die Leuchtverteilung unterschiedliche Lichtbündel umfasst, die sich an dem Wellenleiter (400) überlappen.

6. Vorrichtung (100) nach einem der vorherigen Ansprüche, wobei die Vorrichtung (100) eine Lichtquellenanordnung umfasst, die eingerichtet ist, das Licht bereitzustellen, wobei die Lichtquellenanordnung mindestens eines der folgenden Elemente umfasst:

zwei Lichtquellen, die eingerichtet sind Licht in unterschiedlichen Richtungen und/oder in unterschiedlichen Wellenlängenbereichen und/oder an unterschiedliche Beleuchtungspositionen des mindestens einen Einkopplungselements (440; 441-443) bereitzustellen,
einen Strahlteiler (450),
einen Scanspiegel (460),
ein schaltbares Element (470).

7. Vorrichtung (100) nach einem der Ansprüche 1 bis 6, wobei die optische Kopplung der ersten Gruppe von Replikationsbereichen (510) und/oder der zweiten Gruppe von Replikationsbereichen (520) eine Reihen-optische-Kopplung umfasst und/oder die optische Kopplung eine Baumstruktur (530) umfasst.

8. Vorrichtung (100) nach einem der vorherigen Ansprüche, wobei die optischen Elemente ferner umfassen: mindestens ein Auskopplungselement, welches eingerichtet ist, Licht aus dem Lichtwellenleiter auszukoppeln.

9. Vorrichtung (100) nach Anspruch 8, wobei das mindestens eine Auskopplungselement und/oder das mindestens eine Einkopplungselement (440; 441-442) ein oder mehrere weitere optische Elemente umfassen, ausgewählt aus einer Gruppe umfassend: eine Linse, ein Prisma, ein Oberflächengitter, ein Polarisationsfilter.

10. Vorrichtung (100) nach einem der vorherigen Ansprüche und Anspruch 4, wobei die Leuchtverteilung (200) so eingerichtet ist, dass eine Vielzahl von Strahlen (285) von unterschiedlichen Bereichen des Lichtwellenleiters derart abgegeben werden, sodass das abgegebene Licht effektiv fokussiert und/oder effektiv defokussiert ist.

11. Vorrichtung (100) nach Anspruch 10, wobei die Vielzahl von Strahlen (285) kollimiert sind und/oder in diskreten Winkelbereichen aus dem Lichtleiter abgegeben werden.

12. Vorrichtung (100) nach einem der Ansprüche 8 bis 11, wobei das mindestens eine Auskopplungselement mindestens ein weiteres optisches Element umfasst, welches eingerichtet ist, um ein Muster des ausgekoppelten Lichts zu erzeugen.

13. Vorrichtung (100) nach einem der vorherigen Ansprüche, wobei mindestens eines der optischen Elemente und/oder das mindestens eine weitere optische Element ausgewählt ist aus: diffraktivem Element, schaltbarem diffraktivem Element, Volumenhologramm.

14. Vorrichtung (100) nach einer der vorherigen Ansprüche, wobei das mindestens eine Einkopplungselement (440; 441-443) eingerichtet ist, ein Einkoppeln basierend auf einer Charakteristik des Lichts vorzunehmen und wobei die Replikationsbereiche eingerichtet sind, für mindestens zwei verschiedene Charakteristiken des Lichts mindestens zwei verschiedene zugehörige Leuchtverteilungen (200) zu erzeugen.

15. Vorrichtung (100) nach einem der Ansprüche 1 bis 14, wobei der Lichtwellenleiter (400) eine erste und eine zweite Seite aufweist und wobei die Leuchtverteilung (200) eine erste Leuchtverteilung (200) auf der ersten und eine zweite Leuchtverteilung (200) auf der zweiten Seite des Lichtwellenleiters (400) umfasst.

16. Lichtwellenleitersystem mit einer Vielzahl von Vorrichtungen (100) jeweils gemäß einem der vorherigen Ansprüche, wobei die Vielzahl von Lichtwellenleitern (400) einen gemeinsamen Lichtwellenleiter (400) mit einer Auskopplungsfläche aufweisen,
wobei der gemeinsame Lichtwellenleiter (400) mindestens eine Aussparung (420) mit einer Aussparungsfläche in der Auskopplungsfläche aufweist und wobei die Vielzahl von Vorrichtungen so angeordnet ist, dass die Leuchtverteilungen (200) der Vielzahl von Vorrichtungen (100) von mindestens 80 % der Auskopplungsfläche ohne die Aussparungsfläche ausgehen.

17. Flächenleuchte (170), umfassend eine Vorrichtung (100) gemäß einem der Ansprüche 1 bis 15 oder ein Lichtwellenleitersystem nach Anspruch 16, wobei der Lichtwellenleiter (400) in einer Richtung eine Dimension von weniger als 50 $\mu$m aufweist.

**Claims**

1. Device (100) for generating a light distribution (200) for illuminating an object (700), comprising:

an optical waveguide (400) comprising the following optical elements:

at least one input-coupling element (440; 441-443), configured for coupling light (210) into the optical waveguide (400) as a light beam having an associated beam profile (215),

a multiplicity of replication regions (500) for replicating the light beam, each being configured to receive at least one associated input light beam having an input beam profile and to provide a multiplicity of associated output light beams having respective output beam profiles, wherein the multiplicity of replication regions (500) are volume holograms, wherein the volume holograms are arranged straight or obliquely within the optical waveguide (400) and/or are exposed multiple times,

wherein at least one first replication region (501) of the multiplicity of replication regions (500) is optically coupled (600) to a second replication region (502) of the multiplicity of replication regions (500) so that the second replication region is configured to receive at least one of the multiplicity of associated output light beams (310) of the first replication region as the associated input light beam of the second replication region (305), and

wherein the first replication region is optically coupled (600) to the at least one input-coupling element (440; 441-443) to receive the light beam as the associated input light beam (300) of the first replication region (501),

wherein the device (100) is configured to couple emission light (610) from a set of the multiplicity of replication regions out of the optical waveguide (400) to provide the light distribution (200),

wherein the multiplicity of replication regions (500) comprises a first group of replication regions (510), each being optically coupled to one another, and wherein the replication regions of the first group of replication regions (510) are each configured for:

providing at least one first associated output beam of the multiplicity of output light beams to another replication region of the first group of replication regions (510), and

not providing at least one second associated output beam of the multiplicity of output light beams to another replication region of the first group of replication regions (510) to obtain a set of emission beams (320) of the first group of replication regions (510), and

wherein the multiplicity of replication regions (500) comprises a second group of replication regions (520), each being optically coupled to one another and of which a subset is configured to receive the set of emission beams (320) of the first group of replication regions (510) as the respective input light beams.

2. Device (100) according to Claim 1, wherein the optical waveguide (400) is configured to receive the light with a first modulation (215), wherein the device (100) is configured such that the light distribution (200) has a second modulation (218), wherein the second modulation (218) has a greater number of extreme values than the first modulation (216).

3. Device (100) according to Claim 1 or Claim 2, wherein the device (100) does not comprise a spatial light modulator which is configured to modulate light to be coupled into the optical waveguide based on data.

4. Device (100) according to any one of the preceding claims, wherein at least a subset of the multiplicity of replication regions provides a partial light distribution (200) of the light distribution (200), which has effective focusing.

5. Device (100) according to any one of the preceding claims, wherein the light distribution comprises different light beams that overlap on the waveguide (400).

6. Device (100) according to any one of the preceding claims, wherein the device (100) comprises a light source arrangement which is configured to provide the light, wherein the light source arrangement comprises at least one of the following elements:

two light sources, which are configured to provide light in different directions and/or in different wavelength ranges and/or at different illumination positions of the at least one input-coupling element (440; 441-443),
a beam splitter (450),
a scanning mirror (460),
a switchable element (470).

7. Device (100) according to any one of Claims 1 to 6, wherein the optical coupling of the first group of replication regions (510) and/or the second group of replication regions (520) comprises a series optical coupling and/or the optical coupling comprises a tree structure (530).

8. Device (100) according to any one of the preceding claims, wherein the optical elements further comprise:

at least one output-coupling element, which is configured to couple light out of the optical waveguide.

9. Device (100) according to Claim 8, wherein the at least one output-coupling element and/or the at least one input-coupling element (440; 441-442) comprise one or more further optical elements, selected from a group comprising: a lens element, a prism, a surface grating, a polarization filter.

10. Device (100) according to any one of the preceding claims and Claim 4, wherein the light distribution (200) is configured such that a multiplicity of rays (285) from different regions of the optical waveguide are emitted such that the emitted light is effectively focused and/or effectively defocused.

11. Device (100) according to Claim 10, wherein the multiplicity of rays (285) are collimated and/or are emitted in discrete angle ranges from the light guide.

12. Device (100) according to any one of Claims 8 to 11, wherein the at least one output-coupling element comprises at least one further optical element, which is configured to generate a pattern of the output-coupled light.

13. Device (100) according to any one of the preceding claims, wherein at least one of the optical elements and/or the at least one further optical element is selected from: diffractive element, switchable diffractive element, volume hologram.

14. Device (100) according to any one of the preceding claims, wherein the at least one input-coupling element (440; 441-443) is configured to perform input-coupling based on a characteristic of the light and wherein the replication regions are configured to generate at least two different associated light distributions (200) for at least two different characteristics of the light.

15. Device (100) according to any one of Claims 1 to 14, wherein the optical waveguide (400) has a first and a second side and wherein the light distribution (200) comprises a first light distribution (200) on the first side and a second light distribution (200) on the second side of the optical waveguide (400).

16. Optical waveguide system having a multiplicity of devices (100) each according to any one of the preceding claims, wherein the multiplicity of optical waveguides (400) have one common optical waveguide (400) having an output-coupling surface,
wherein the common optical waveguide (400) has at least one cutout (420) with a cutout surface in the output-coupling surface and wherein the multiplicity of devices are arranged such that the light distributions (200) of the multiplicity of devices (100) come from at least 80% of the output-coupling surface without the cutout surface.

17. Surface luminaire (170) comprising a device (100) according to any one of Claims 1 to 15 or an optical waveguide system according to Claim 16, wherein the optical waveguide (400) has in one direction a dimension of less than 50 μm.

**Revendications**

1. Dispositif (100) permettant de produire une distribution lumineuse (200) pour éclairer un objet (700), comprenant :
un guide d'ondes optiques (400), comprenant les éléments optiques suivants :

au moins un élément d'injection (440 ; 441-443) conçu pour injecter de la lumière (210) dans le guide d'ondes optiques (400) sous forme de faisceau de lumière avec un profil de faisceau (215) associé,
une pluralité de zones de réplication (500) pour répliquer le faisceau de lumière, respectivement conçues pour recevoir au moins un faisceau de lumière d'entrée associé avec un profil de faisceau d'entrée et pour fournir une pluralité de faisceaux de lumière de sortie associés avec des profils de faisceau de sortie respectifs, dans lequel la pluralité de zones de réplication (500) sont des hologrammes en volume, les hologrammes en volume étant disposés de manière droite ou oblique à l'intérieur du guide d'ondes optiques (400) et/ou étant éclairés plusieurs fois,
dans lequel au moins une première zone de réplication (501) de la pluralité de zones de réplication (500) est couplée optiquement (600) à une deuxième zone de réplication (502) de la pluralité de zones de réplication (500) de sorte que la deuxième zone de réplication est conçue pour recevoir au moins l'un de la pluralité de faisceaux de lumière de sortie (310) associés de la première zone de réplication en tant que faisceau de lumière d'entrée associé de la deuxième zone de réplication (305), et

dans lequel la première zone de réplication est couplée optiquement (600) audit au moins un élément d'injection (440 ; 441-443) pour recevoir le faisceau de lumière en tant que faisceau de lumière d'entrée (300) associé de la première zone de réplication (501),

dans lequel le dispositif (100) est conçu pour déclencher du guide d'ondes optiques (400) la lumière dégagée (610) d'une quantité de la pluralité de zones de réplication afin de fournir la distribution lumineuse (200),

dans lequel la pluralité de zones de réplication (500) comprend un premier groupe de zones de réplication (510) qui sont respectivement couplées optiquement ensemble, et dans lequel les zones de réplication du premier groupe de zones de réplication (510) sont respectivement conçues pour :

fournir au moins un premier faisceau de sortie associé de la pluralité de faisceaux de lumière de sortie à une autre zone de réplication du premier groupe de zones de réplication (510), et

ne pas fournir au moins un deuxième faisceau de sortie associé de la pluralité de faisceaux de lumière de sortie à une autre zone de réplication du premier groupe de zones de réplication (510) afin d'obtenir une quantité de faisceaux dégagés (320) du premier groupe de zones de réplication (510), et

dans lequel la pluralité de zones de réplication (500) comprend un deuxième groupe de zones de réplication (520) qui sont respectivement couplés optiquement ensemble et dont un sous-ensemble est conçu pour recevoir la quantité de faisceaux dégagés (320) du premier groupe de zones de réplication (510) en tant que faisceaux de lumière d'entrée respectifs.

2. Dispositif (100) selon la revendication 1, dans lequel le guide d'ondes optiques (400) est conçu pour recevoir la lumière avec une première modulation (215), dans lequel le dispositif (100) est conçu de telle sorte que la distribution lumineuse (200) présente une deuxième modulation (218), dans lequel la deuxième modulation (218) présente un nombre de valeurs extrêmes supérieur à celui de la première modulation (216).

3. Dispositif (100) selon la revendication 1 ou la revendication 2, dans lequel le dispositif (100) ne comprend aucun modulateur de lumière dans l'espace qui est conçu pour moduler la lumière à injecter dans le guide d'ondes optiques sur la base de données.

4. Dispositif (100) selon l'une quelconque des revendications précédentes, dans lequel au moins un sous-ensemble de la pluralité de zones de réplication fournit une distribution lumineuse partielle (200) de la distribution lumineuse (200) qui présente une focalisation effective.

5. Dispositif (100) selon l'une quelconque des revendications précédentes, dans lequel la distribution lumineuse comprend différents faisceaux de lumière qui se chevauchent au niveau du guide d'ondes (400).

6. Dispositif (100) selon l'une quelconque des revendications précédentes, dans lequel le dispositif (100) comprend un agencement de sources de lumière qui est conçu pour fournir la lumière, l'agencement de sources de lumière comprenant au moins l'un des éléments suivants :

deux sources de lumière qui sont conçues pour fournir de la lumière dans différentes directions et/ou dans différentes plages de longueurs d'onde et/ou dans différentes positions d'éclairage dudit au moins un élément d'injection (440 ; 441-443),

un séparateur de faisceau (450),

un miroir de balayage (460),

un élément commutable (470).

7. Dispositif (100) selon l'une quelconque des revendications 1 à 6, dans lequel le couplage optique du premier groupe de zones de réplication (510) et/ou du deuxième groupe de zones de réplication (520) comprend un couplage optique en série et/ou le couplage optique comprend une structure arborescente (530).

8. Dispositif (100) selon l'une quelconque des revendications précédentes, dans lequel les éléments optiques comprennent en outre :

au moins un élément de déclenchement qui est conçu pour déclencher de la lumière du guide d'ondes optiques.

9. Dispositif (100) selon la revendication 8, dans lequel au moins un élément de déclenchement et/ou ledit au moins un élément d'injection (440 ; 441-442) comprennent un ou plusieurs autres éléments optiques, sélectionnés dans un groupe comprenant : une lentille, un prisme, un réseau de surface, un filtre de polarisation.

10. Dispositif (100) selon l'une quelconque des revendications précédentes et selon la revendication 4, dans lequel la distribution lumineuse (200) est conçue de telle sorte qu'une pluralité de faisceaux (285) est dégagée par différentes zones du guide d'ondes optiques de telle sorte que la lumière dégagée est effectivement focalisée et/ou effectivement défocalisée.

11. Dispositif (100) selon la revendication 10, dans lequel la pluralité de faisceaux (285) est collimatée et/ou est dégagée du guide de lumière dans des plages angulaires discrètes.

12. Dispositif (100) selon l'une quelconque des revendications 8 à 11, dans lequel ledit au moins un élément de déclenchement comprend au moins un autre élément optique qui est conçu pour produire un motif de la lumière déclenchée.

13. Dispositif (100) selon l'une quelconque des revendications précédentes, dans lequel au moins l'un des éléments optiques et/ou ledit au moins un autre élément optique sont sélectionnés parmi : un élément diffractif, un élément commutable, un hologramme en volume.

14. Dispositif (100) selon l'une quelconque des revendications précédentes, dans lequel ledit au moins un élément d'injection (440 ; 441-443) est conçu pour procéder à l'injection sur la base d'une caractéristique de la lumière, et dans lequel les zones de réplication sont conçues pour produire au moins deux distributions lumineuses (200) associées différentes pour au moins deux caractéristiques différentes de la lumière.

15. Dispositif (100) selon l'une quelconque des revendications 1 à 14, dans lequel le guide d'ondes optiques (400) présente un premier et un deuxième côté, dans lequel la distribution lumineuse (200) comprend une première distribution lumineuse (200) sur le premier côté et une deuxième distribution lumineuse (200) sur le deuxième côté du guide d'ondes optiques (400).

16. Système de guide d'ondes optiques, comprenant une pluralité de dispositifs (100) respectivement selon l'une quelconque des revendications précédentes, dans lequel la pluralité de guides d'ondes optiques (400) présente un guide d'ondes optiques commun (400) pourvu d'une surface de déclenchement, dans lequel le guide d'ondes optiques commun (400) présente au moins un évidement (420) ayant une surface d'évidement dans la surface de déclenchement, et dans lequel la pluralité de dispositifs est disposée de telle sorte que les distributions lumineuses (200) de la pluralité de dispositifs (100) émanent d'au moins 80 % de la surface de déclenchement sans la surface d'évidement.

17. Panneau lumineux (170), comprenant un dispositif (100) selon l'une quelconque des revendications 1 à 15 ou un système de guide d'ondes optiques selon la revendication 16, dans lequel le guide d'ondes optiques (400) présente une dimension de moins de 50 $\mu$m dans une direction.

Fig. 1A

Fig. 1B

Fig. 2

100

440

501

Legende:

□ 500

⬚ 440

530

Fig. 3

Fig. 5

Fig. 4

Fig. 6

Fig. 7B

Fig. 7A

Legende:
□ 500

**Fig. 8**

Legende:
□ 500
⬚ 440

**Fig. 9**

EP 3 946 000 B1

Fig. 11

Fig. 10

Legende:

◯ 500

⬭ 440

100

400

Fig. 12

Fig. 13A

Fig. 13B

EP 3 946 000 B1

Fig. 14

Fig. 15

Fig. 16

Fig. 18

Legende:
440
500
610
600

Fig. 17

Fig. 19

EP 3 946 000 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8320032 B2
- US 20160231568 A1
- US 2002149924 A1
- DE 102014101219 A1
- DE 102016116311 A1
- DE 102014112242 A1
- DE 102011102355 A1